# NOUVEAU FASCICULE DE BREVET EUROPEEN

(11) **EP 0 521 748 B2**
(45) Date de publication et mention de la décision concernant l'opposition: **09.03.2005**
(45) Mention de la délivrance du brevet: 24.04.1996
(21) Numéro de dépôt: 92401690.0
(22) Date de dépôt: 17.06.1992
(51) Int. Cl.: A61K 7/06, A61K 7/48

(54) **Composition de lavage et/ou de conditionnement des matières kératiniques, contenant une silicone et un polymère amphotère dérivé de diallyldialkylammonium et d'un monomère anionique**
Mittel zum Waschen und/oder Konditionieren von Keratinmaterial, das ein Silikon und ein amphoteres Polymer aus Diallyldialkylammonium und einem anionischen Monomer enthält
Washing and/or conditioning composition for keratinous material containing a silicone and an amphoteric polymer derivative of diallyldialkylammonium and an anionic monomer

(30) Priorité: 21.06.1991 FR 9107677
(43) Date de publication de la demande: 07.01.1993
(73) Titulaire: L'OREAL, 75008 Paris (FR)
(72) Inventeur: Dubief, Claude, F-78150 Le Chesnay (FR); Cauwet, Danièle, F-75011 Paris (FR)
(74) Mandataire: Casalonga, Axel

(56) Documents cités:
- EP-A- 0 203 750
- EP-A- 0 269 243
- EP-A- 0 392 320
- EP-A- 0 401 867
- FR-A- 2 548 019
- GB-A- 2 058 103

## Description

La présente invention a pour objet des compositions de lavage et/ou de conditionnement des matières kératiniques et plus particulièrement des cheveux, renfermant une silicone et un polymère amphotère dérivé de diallyldialkylammonium et d'un monomère anionique et aux procédés de traitement des matières kératiniques mettant en oeuvre de telles compositions.

Il est bien connu que les cheveux sont sensibilisés ou fragiles à des degrés divers par l'action d'agents atmosphériques ainsi que par l'action de différents traitements cosmétiques tels que permanentes, défrisages, teintures ou décolorations. Les cheveux deviennent alors difficile à démêler et à coiffer. De plus, ils deviennent rêches au toucher.

On a déjà utilisé, dans des compositions de lavage, des silicones qui apportent douceur, brillance, légèreté et peuvent faciliter le démêlage.

On a également déjà utilisé dans le passé des polymères amphotères dans des compositions de traitement des cheveux, tels que par exemple des copolymères d'octylacrylamide/acrylatelbutylaminoéthylméthacrylate, les copolymères des méthacrylates d'alkyle en C₁-C₁₈/méthacrylate de carboxyméthyldiméthylammoniométhyle ou les dérivés du chitosane. EP-A-0203750 décrit une composition de nettoyage de la peau très douce, comprenant un agent de surface, un agent humectant, un polymère et un savon.

La demanderesse a constaté de façon surprenante qu'en associant dans une composition de traitement des matières kératiniques une silicone et un copolymère dérivé de diallyldialkylammonium et d'un monomère anionique et plus particulièrement d'acide acrylique, il était possible d'obtenir, lorsqu'elle était appliquée en particulier sur des cheveux, d'excellentes propriétés de démêlage et de douceur par rapport à des cheveux traités avec des compositions ne renfermant que la silicone, ou le polymère amphotère, seul.

Les cheveux ainsi traités sont particulièrement faciles à démêler, doux et légers. Ifs sont par ailleurs disciplinés, se mettent facilement en forme et ont plus de coiffant, cet effet est particulièrement intéressant lorsque les cheveux sont abîmés, sensibilisés ou dans le traitement des cheveux fins.

L'objet de l'invention est donc constitué par une composition de traitement des matières kératiniques renfermant au moins une silicone et un polymère amphotère dérivé de diallyldialkylammonium et d'un monomère anionique.

Un autre objet de l'invention est constitué par un procédé de traitement des matières kératiniques mettant en oeuvre une telle composition.

L'invention a également pour objet l'utilisation de l'association d'un polymère dérivé de diallyldialkylammonium et d'un monomère anionique et de silicones pour le traitement des matières kératiniques et en particulier pour conférer des propriétés de douceur et de démêlage améliorées aux cheveux.

D'autres objets de l'invention apparaîtront à la lecture de la description et des exemples qui suivent.

Les compositions de lavage et de conditionnement des matières kératiniques, conformes à l'invention, sont essentiellement caractérisées par le fait qu'elles contiennent dans un milieu aqueux :
- au moins un agent tensio-actif possédant des propriétés détergentes dans des proportions comprises entre 5 et 50% en poids par rapport au poids total de la composition.
- au moins un copolymère de diallyldialkylammonium et d'un monomère anionique.
et ne contenant pas d'agents de mise en suspension constitués par des alcools ayant 27 à 44 atomes de carbone et comprenant un ou deux groupements ether et/ou thioethers ou sulfoxyde.

Les agents tensio-actifs utilisés dans les compositions de lavage et de conditionnement, conformes à l'invention, sont connus en eux-mêmes et sont choisis parmi les agents tensio-actifs anioniques, amphotères, zwittérioniques, non-ioniques ou leurs mélanges, ayant des propriétés détergentes.

Parmi les agents tensio-actifs anioniques, on peut citer les sels alcalins, les sels d'ammonium, les sels d'amines, les sels d'aminoalcools, les sels de magnésium des composés suivants : les alkyléthersulfates, alkylamidoéthersulfates, alkylarylpolyéthersulfates, monoglycérides sulfates; les alkylsulfonates, alkylamides sulfonates, alkylarylsulfonates, oléfines sulfonates, paraffines suffonates; les alkylsulfosuccinates, les alkyléthersulfosuccinates, les alkylamides sulfosuccinates; les alkylsulfosuccinamates; les alkylsulfoacétates; les alkylétherphosphates; les acylsarcosinates, les N-acyltaurates.

Le radical alkyle ou acyle de ces différents composés est généralement constitué par une chaîne carbonée comportant de 12 à 20 atomes de carbone.

Parmi les agents tensio-actifs anioniques, on peut également citer les sels d'acides gras tels que les sels des acides oléique, ricinoléique, palmitique, stéarique; les acides d'huile de coprah ou d'huile de coprah hydrogénée; les acyllactylates, dont le radical acyle comporte de 8 à 20 atomes de carbone.

On peut également utiliser des agents tensio-actifs faiblement anioniques, tels que les acides éthers carboxyliques polyoxyalkylénés, en particulier ceux comportant 2 à 50 groupements oxyde d'éthylène.

Les agents tensio-actifs non-ioniques sont plus particulièrement choisis parmi les alcools ou les α-diols ou les alkylphénols ou les acides gras polyéthoxylés, polypropoxylés ou polyglycérolés, à chaîne grasse comportant 8 à 18 atomes de carbone, le nombre de groupements oxyde d'éthylène ou oxyde de propylène étant compris entre 2 et 50 et le nombre de groupements glycérol étant compris entre 2 et 30.

On peut également citer les copolymères d'oxydes d'éthylène et de propylène; les condensats d'oxydes d'éthylène et de propylène sur des alcools gras; les amides gras polyéthoxylés ayant de préférence 2 à 30 moles d'oxyde d'éthylène; les amides gras polyglycérolés comportant de préférence 1 à 5 groupements glycérol et en particulier 1,5 à 4; les amines grasses polyéthoxylées ayant de préférence 2 à 30 moles d'oxyde d'éthylène; les esters d'acides gras du sorbitan oxyéthylénés ayant de préférence 2 à 30 moles d'oxyde d'éthylène; les esters d'acides gras de sucrose, les esters d'acides gras du polyéthyièneglycol, les esters d'acides gras de glycols, les alkylpolyglycosides, les oxydes d'amines tels que les oxydes d'alkyl(C₁₀-C₁₄) amines ou de N-acylamidopropylmorpholine.

Les agents tensio-actifs amphotères ou zwittérioniques préférés sont les dérivés d'amines secondaires ou tertiaires aliphatiques, dans lesquels le radical aliphatique est une chaîne linéaire ou ramifiée comportant 8 à 18 atomes de carbone et qui contient au moins un groupe anionique hydrosolubilisant carboxylate, sulfonate, sulfate, phosphate ou phosphonate; les alkyl(C₈-C₂₀)bétaïnes, les sulfobétaïnes, les alkyl(C₈-C₂₀)amidoalkyl(C₁-C₆)bétaïnes ou les alkyl (C₈-C₂₀)amidoalkyl(C₁-C₆)sulfobétaïnes.

Parmi les dérivés d'amines, on peut citer les produits vendus sous la dénomination MIRANOL, tels que décrits dans les brevets US-A-2.528.378 et 2.781.354 et classés dans le dictionnaire CTFA, 3ème édition, 1982, sous les dénominations d'Amphocarboxyglycinates et Amphocarboxypropionates.

Les agents tensio-actifs sont utilisés dans les compositions conformes à l'invention dans des proportions suffisantes pour conférer un caractère détergent à la composition et sont comprises de préférence entre 5 et 50% en poids par rapport au poids total de la composition et en particulier entre 8 et 35%.

Les silicones, utilisées conformément à la présente invention, sont des polyorganosiloxanes insolubles dans les milieux aqueux, pouvant se présenter sous forme d'huiles, de cires, de gommes ou de résines.

Les organopolysiloxanes sont définis plus en détail dans l'ouvrage de Walter NOLL "Chemistry and Technology of Silicones" (1968) Academic-Press.

Les polysiloxanes utilisés, conformément à l'invention, sont choisis parmi les silicones volatiles possédant un point d'ébullition compris entre 60°C et 260°C, ou bien les silicones non volatiles choisies en particulier parmi les polyalkylsiloxanes, les polyaryl siloxanes, les polyalkylarylsiloxanes, les gommes et résines de silicones, les polysiloxanes organomodifiés ainsi que leurs mélanges.

Les silicones volatiles peuvent être choisies parmi :
(i) les silicones cycliques comportant de 3 à 7 atomes de silicium et de préférence 4 à 5. Il s'agit, par exemple, de l'octaméthylcyclo tétrasiloxane vendu sous le nom de VOLATILE SILICONE 7207 par UNION CARBIDE ou SILBIONE 70045 V 2 par RHONE POULENC, le décaméthylcyctopentasiloxane vendu sous le nom de VOLATILE SILICONE 7158 par UNION CARBIDE, SILBIONE 70045 V 5 par RHONE POULENC, ainsi que leurs mélanges.
   On peut citer également les cyclocopolymères du type diméthylsiloxane/méthylalkylsiloxane, tel que la SILICONE VOLATILE FZ 3109 vendue par la Société UNION CARBIDE, qui est un cyclopolymère diméthylsiloxane/méthyloctylsiloxane.
   On peut utiliser par ailleurs les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthy)sitytoxy)bis-néopentane;
(ii) les silicones volatiles linéaires ayant 2 à 9 atomes de silicium et possédant une viscosité inférieure ou égale à 5.10⁻⁶ m²/s à 25°C. Il s'agit, par exemple, de l'hexaméthyldisiloxane vendu sous la dénomination SILBIONE 70 041 V 0,65 par la Société RHONE POULENC, du décaméthyltétrasiloxane vendu sous la dénomination SH 200 par la Société TORAY SILICONE ou par les polyméthylphénylsiloxanes volatils tels que le produit SILICONOL AS vendu par la Société WACKER. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Les silicones non volatiles sont choisies notamment parmi les polyalkylsiloxanes. On peut citer principalement les polydiméthyl siloxanes linéaires à groupements terminaux triméthylsilyle de viscosité 5.10⁻⁶ à 2,5 m²/s à 25°C et de préférence 10-⁵ à 1 m²/s, comme par exemple et à titre non limitatif:
. les huiles SILBIONE des séries 47 et 70 047 commercialisées par RHONE POULENC, telles que l'huile 47 V 500.000,
. les huiles de la série 200 de la Société DOW CORNING, · les huiles VISCASIL de la GENERAL ELECTRIC et certaines huiles des séries SF de la GENERAL ELECTRIC (SF 96, SF 18).

On cite également les polydiméthylsiloxanes linéaires à groupements terminaux diméthylsilanol, tels que les huiles de la série 48 de RHONE POULENC.

Dans cette classe de polyalkylsiloxanes, on peut également mentionner les cires de polyalkylsiloxanes vendues par la Société GOLDSCHMIDT sous les dénominations ABIL WAX 9800 et ABIL WAX 9801, qui sont des polyalkyl(C₁-C₂₀)-siloxanes.

Parmi les polyalkylarylsiloxanes, on peut citer les polydiméthyl méthylphénylsiloxanes, les polyméthylphénylsiloxanes, les poly diméthyldiphénylsiloxanes linéaires et/ou ramifiés, de viscosité 10-⁵ à 5.10⁻² m²/s à 25°C, tels que, par exemple :
. les huiles des séries RHODORSIL 70 633 et 763 de RHONE POULENC,
. les huiles SILBIONE de la série 70 641 de RHONE POULENC,
. l'huile DC 556 COSMETIC GRAD FLUID de DOW CORNING,
. les silicones de la série PK de BAYER, telles que la PK20,
. les silicones des séries PN, PH de BAYER, comme les PN 1000 et PH 1000,
. certaines huiles des séries SF de GENERAL ELECTRIC, telles que les SF 1250, SF 1265, SF 1154, SF 1023.

Les gommes de silicones, conformes à la présente invention, sont des polydiorganosiloxanes de fortes masses moléculaires, comprises entre 200.000 et 1.000.000. utilisées seules ou en mélange dans un solvant choisi parmi les silicones volatiles telles que définies ci-dessus, les huiles poly diméthylsiloxanes (PDMS), les huiles poly phénylméthylsiloxanes (PPMS), les isoparaffines, le chlorure de méthylène, le pentane, le dodécane, le tridécane, le tétradécane ou leurs mélanges.

On cite, par exemple, les gommes suivantes :
- poly[(diméthylsiloxane)/(méthylvinylsiloxane)],
- poly[(diméthylsiloxane)l(diphénylsiloxane)],
- poly[(diméthylsiloxane)/(phény)méthyisitoxane)],
- poly[(diméthylsiloxane)/(diphénylsiloxane)/méthylvinylsiloxane)].

On peut citer, par exemple, à titre non limitatif, les mélanges suivants :
· les mélanges formés à partir d'un polydiméthylsiloxane hydroxylé en bout de chaîne (DIMETHICONOL selon la nomenclature CTFA) et d'un polydiméthylsiloxane cyclique (CYCLOMETHICONE selon la nomenclature CTFA), tel que le produit Q2 1401 vendu par la Société DOW CORNING,
· les mélanges formés à partir d'une gomme polydiméthylsiloxane avec une silicone cyclique, tel que le produit SF 1214 Silicone Fluid de la Société GENERAL ELECTRIC (qui est une gomme SE 30, correspondant à une diméthicone, ayant un poids moléculaire de 500.000 solubilisée dans la silicone SF 1202 Silicone Fluid (correspondant au décaméthylcyclopentasiloxane)),
· les mélanges de deux PDMS de viscosités différentes, notamment d'une gomme PDMS et d'une huile PDMS, tels que les produits SF 1236 et CF 1241 de la Société GENERAL ELECTRIC. Le produit SF 1236 est le mélange d'une gomme SE 30 définie ci-dessus d'une viscosité de 20 m²/s et d'une huile SF 96 d'une viscosité de 5. 10⁻⁶ m²/s (15% de gomme SE 30 et 85% d'huile SF 96).

Le produit CF 1241 est le mélange d'une gomme SE 30 (33%) et d'une PDMS (67%) de viscosité 10⁻³ m²/s.

Les résines d'organopolysiloxanes utilisables conformément à l'invention, sont des systèmes siloxaniques réticulés renfermant les unités R'₂SiO_{2/2}, R'SiO_{3/2} et SiO_{4/2}, dans lesquelles R' représente un groupement hydrocarboné possédant 1 à 6 atomes de carbone ou un groupement phényle. Parmi ces produits, ceux particulièrement préférés sont ceux dans lesquels R' désigne un radical alkyle inférieur ou un radical phényle.

Parmi ces résines, on peut citer le produit vendu sous la dénomination DOW CORNING 593 ou ceux vendus sous les dénominations SILICONE FLUID SS 4230 et SS 4267 par la Société GENERAL ELECTRIC et qui sont des "diméthyl/triméthylpoly siloxane"

Les silicones organomodifiées sont des silicones définies ci-dessus et comportant dans leur structure un ou plusieurs groupements organo-fonctionnels directement fixés sur la chaîne siloxanique ou fixés par l'intermédiaire d'un radical hydrocarboné.

Parmi ces silicones organomodifiées, on peut citer, par exemple, les silicones comportant :
1- des groupements polyéthylèneoxy et/ou polypropylèneoxy, comportant éventuellement des groupes alkyles, tels que:
   · le produit dénommé diméthicone copolyol vendu par la Société DOW CORNING sous les dénominations DC 1248, et l'alkyl(C₁₂) méthicone copolyol vendue par la Société DOW CORNING sous la dénomination Q2 5200,
   · les huiles SILWET L 722, L 7500, L 77, L 711 de la Société UNION CARBIDE,
   · le mélange de diméthicone copolyol et de cyclométhicone tels que le produit vendu sous la dénomination Q2-3225C par la Société DOW CORNING,
2- des groupements aminés substitués ou non comme les produits vendus sous la dénomination GP4 Silicone Fluid et GP 7100 par la Société GENESEE ou les produits vendus sous les dénominations Q2 8220, X2 8200 et DC 929 ou Q2 7224 par la Société DOW CORNING. Les groupements aminés substitués sont en particulier des groupements aminoalkyle(C₁-C₄),
3- des groupements thiols comme dans les GP 72 A et GP 71 de GENESEE ou dans le produit SLM 50253/5 de la Société WACKER,
4- des groupements carboxylates comme dans les produits décrits dans le brevet EP-A-186.507 de la Société CHISSO CORPORATION,
5 - des groupements alkoxylés comme les produits vendus sous la dénomination SILICONE COPOLYMER F-755 par SWS SILICONES, et ABIL WAX 2428, 2434 et 2440 par la Société GOLDSCHMIDT,
6- des groupements hydroxylés, comme les polyorganosiloxanes à fonction hydroxyalkyle, décrits dans la demande de brevet français n° FR-85.16.334.
   On cite, par exemple, le produit 71615 V 300 vendu par la Société RHONE POULENC.
7- des groupements acyloxyalkyle, comme par exemple les polyorgano siloxanes décrits dans la demande de brevet FR-A-2.641.185.
8- des groupements anioniques de type carboxylique tels que les groupements alkylcarboxyliques comme ceux présents dans le produit X-22-3701E de la Société SHIN-ETSU ou dans le produit Silicone Fluid FZ 3703 de la Société UNION CARBIDE; 2-hydroxyalkylsulfonate; 2-hydroxyalkylthiosulfate tels que les produits vendus par la Société GOLDSCHMIDT sous les dénominations "ABIL S201" et "ABIL S255".
9- des groupements hydroxyacylamino comme les polyorganosiloxanes décrits dans la demande EP-A-342834.

On peut citer à titre d'exemple le produit Q2 8413 de la Société DOW-CORNING, répondant à la formule :

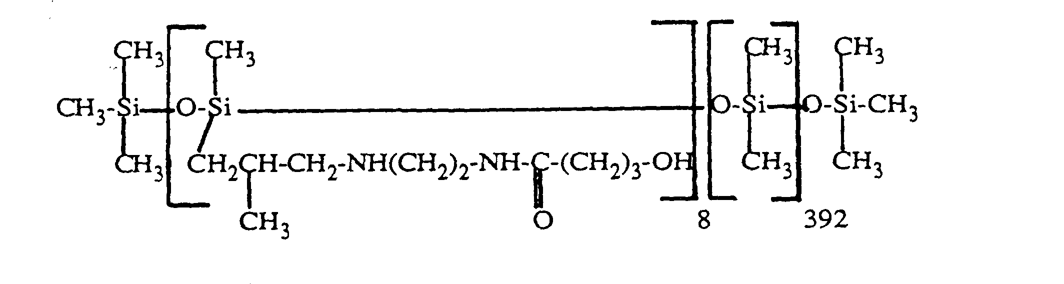

Les polyorganosiloxanes plus particulièrement préférés, conformément à l'invention, sont :
· les huiles de silicone de forte viscosité comprise entre 0,2 et 2,5 m²/s à 25°C, telles que les huiles 47 V 500.000 de la Société RHONE POULENC,
· les mélanges d'organopolysiloxanes et de silicones cycliques, tels que le produit Q2 1401 vendu par la Société DOW CORNING,
· les mélanges de deux PDMS de viscosités différentes, tels que le produit vendu par la Société GENERAL ELECTRIC sous la dénomination CF 1241,
· les silicones comportant des groupements hydroxyacylamino.

Les silicones insolubles dans l'eau, utilisées dans les compositions conformes à l'invention, sont présentes dans des proportions comprises entre 0,2 et 30% et de préférence entre 0,4 et 15% en poids par rapport au poids total des compositions.

Le polymère dérivé de diallyldialkylammonium et d'un monomère anionique utilisé conformément à l'invention, est en particulier un polymère comportant environ 60 à environ 99% en poids d'unités dérivées d'un monomère de diallyldialkylammonium quaternaire dans lequel les groupements alkyle sont choisis indépendamment parmi les groupements alkyle ayant 1 à 18 atomes de carbone et dans lequel l'anion est dérivé d'un acide ayant une constante d'ionisation supérieure à 10⁻¹³ et 1 à 40% en poids de ce polymère, d'un monomère anionique choisi parmi les acides acrylique ou méthacrylique, le poids moléculaire de ce polymère étant compris entre environ 50.000 et 10.000.000 déterminé par chromatographie par perméation de gel. De tels polymères sont décrits dans la demande EP-A-269.243.

Les polymères préférés sont entre autres les polymères comportant des groupements alkyle choisis parmi les groupements ayant 1 à 4 atomes de carbone et plus particulièrement des groupements méthyle, éthyle.

Parmi ces polymères, les copolymères de chlorure de diméthyldiallylammonium ou de diéthyldiallylammonium et d'acide acrylique sont particulièrement préférés.

A titre de produits particulièrement préférés, on peut citer le polymère vendu sous la dénomination MERQUAT 280 par la Société CALGON sous forme d'une solution aqueuse à 35% de matière active, ce polymère étant un copolymère de chlorure de diallyldiméthyl ammonium et d'acide acrylique dans les proportions 80/20, la viscosité au viscosimètre Brookfield LVF module 4 étant comprise entre 4000 et 10.000 cps, le poids moléculaire étant environ égal à 1.300.000.

Ce polymère est utilisé dans des proportions en poids par rapport au poids total de la composition compris entre 0,1 et 10% en poids et de préférence entre 0,5 et 5% en poids par rapport au poids total de la composition.

Le pH des compositions est généralement compris entre 2 et 9 et plus particulièrement entre 3 et 8.

Le milieu aqueux peut être constitué uniquement par de l'eau ou un mélange d'eau et d'un solvant cosmétiquement acceptable, tel qu'un alcool inférieur en C₁-C₄ comme l'éthanol, l'isopropanol, le n-butanol; les alkylèneglycols comme l'éthylènegtycol, les éthers de glycol.

Les compositions conformes à l'invention peuvent également contenir des agents de mise en suspension autres que des alcools ayant 27 à 44 atomes de carbone et comportant un ou deux groupements éther et/ou thioéther ou sulfoxyde.

Parmi ces agents, on peut citer plus particulièrement les composés suivants :
a) RX (III)
   dans laquelle R est un radical aliphatique à longue chaîne carbonée, éventuellement interrompue par des atomes d'oxygène, et X est un reste acide carboxylique, sulfurique ou phosphorique ou un radical dérivé d'un acide carboxylique ou d'un amide.
   Ces composés de formule (III) sont choisis parmi ceux dans lesquels :
   (i) R est un radical alkyle ou alcényle en C₁₁-C₂₁
      Xest:
      · un groupement COOA où A est un radical mono ou polyhydroxy alkyle dérivé d'un polyol en C₂-C₃ ou un radical CH₂CH₂SO₃M,
      · un groupement CO(OCH₂CH₂)ₙ)-OH où n a une valeur comprise entre 2 et 150,
      · un groupement 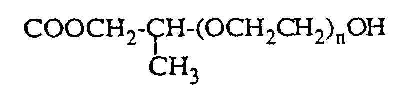 où n a une valeur comprise entre 2 et 150, les fonctions OH libres des groupements définis ci-dessus pouvant être estérifiées par un acide RCOOH où R est un alkyle ou un alcényle en C₁₁-C₂₁,
      · un groupement CONR₁R₂ où R₁ et R₂ représentent hydrogène ou hydroxyalkyle en C₁-C₄, l'un au moins représentant hydroxyalkyle en C₁-C₄,
      · un groupement OSO₃M ou 1/3 PO₄³⊖ M₃ où M représente un métal alcalin, ammonium ou un reste d'alcanolamine en C₁-C₄:
   (ii) R désigne un radical R₃(OC₂H₄)ℓOCH₂ et X désigne un groupement COOM où M a la signification indiquée ci-dessus, R₃ désignant un radical alkyle en C₁₂-C₁₄ et ℓ un nombre entier ou décimal compris entre 2,5 et 10, ou bien R₃ désigne oléyle et ℓ varie de 2 à 9 ou encore R₃ désigne alkyl(C₈-C₉)phényle et ℓ varie de 4 à 8, ou les dérivés dans lesquels R désigne un groupement alkyl(C₁₂-C₁₆) éther et X un groupement CONR₁R₂, dans lequel R₁ et R₂ ont la même signification que celle indiquée ci-dessus;
b) des oxydes de diméthylalkyl(C₁₆-C₂₂)amines;
c) les biopolysaccharides choisis par exemple parmi les gommes de xanthane et les scléroglucanes.

Ces agents de mise en suspension sont utilisés dans les compositions conformes à l'invention, dans des proportions comprises entre 0,1 et 20% en poids et de préférence entre 0,5 et 10% en poids par rapport au poids total de la composition.

Ces compositions peuvent également contenir jusqu'à 3% d'agents nacrants ou opacifiants, tels que des palmitates de sodium ou de potassium, des stéarates ou hydroxystéarates de sodium ou de potassium, le mono- ou distéarate d'éthylèneglycol.

Les compositions conformes à l'invention peuvent également renfermer des agents régulateurs de viscosité, tels que des électrolytes comme le chlorure de sodium ou le xylènesutfonate de sodium, des hydrotropes, des épaissisants comme les dérivés de cellulose, comme par exemple la carboxyméthylcellulose, l'hydroxypropylcellulose, l'hydroxyéthylcellulose, la gomme de guar, les gommes de guar hydroxypropylées.

Ces agents régulateurs de viscosité sont utilisés dans des proportions allant jusqu'à 10% en poids par rapport au poids total de la composition et de préférence inférieure à 5%.

Les compositions selon l'invention peuvent éventuellement renfermer en plus d'autres agents ayant pour effet d'améliorer les propriétés des matières kératiniques, en particulier les propriétés cosmétiques des cheveux, à condition qu'elles n'altèrent pas la stabilité des compositions, tels que des agents tensio-actifs cationiques, des polymères autres que les copolymères de diallyl dialkylammonium et d'un monomère anionique ou des protéines ou encore des silicones solubles dans le milieu.

Les polymères, les tensio-actifs cationiques, les protéines et les silicones utilisés en outre dans les compositions conformes à l'invention, sont utilisés dans des proportions comprises entre 0,05 et 6% et de préférence entre 0,1 et 3% en poids par rapport au poids total de la composition.

Les compositions selon l'invention peuvent enfin renfermer différents adjuvants habituellement utilisés dans les compositions de lavage, tels que des parfums, des conservateurs, des séquestrants, des stabilisateurs de mousse, des agents propulseurs, des colorants, des filtres solaires, des agents acidifiants ou alcalinisants ou d'autres adjuvants selon l'usage envisagé.

Les procédés de lavage et/ou de conditionnement des matières kératiniques et en particulier des cheveux, consistent à appliquer sur ceux-ci une composition telle que définie ci-dessus, cette application étant suivie d'une étape de rinçage.

On peut utiliser entre autre les compositions comme shampooings mais également comme gels douche pour le lavage des cheveux et de la peau, auquel cas ils sont appliqués sur la peau et les cheveux humides et sont rincés après application.

Les exemples qui suivent sont destinés à illustrer l'invention sans pour autant présenter un caractère limitatif.

### EXMEPLE 1

On prépare un shampooing de composition suivante :
- Tensio-actif de type acide éther carboxylique polyoxyéthyléné, de formule :

   R(OCH₂CH₂)ₙ OCH₂COOH

   dans laquelle :
   R = nonylphénol
   n = valeur moyenne de 7
   vendu sous la dénomination AKYPO NP 70
   par la Société CHEM Y à 90% de MA 10,0 g MA
   - Laurylsulfate de triéthanolamine à 40% de MA 10,0 g MA
   - Copolymère de chlorure de diallyl diméthyl
      ammonium et d'acide acrylique, vendu sous
      la dénomination MERQUAT 280 par la
      Société CALGON à 35% de MA 1,0 g MA
   - Polyphénylméthylsiloxane vendu sous la
      dénomination SILBIONE HUILE 70633
      V 30 par la Société RHONE POULENC 2,5 g
   - Diéthanolamide d'acide de coprah 2,5 g
   - Chlorure de sodium 4,0 g
   - Triéthanolamine qs pH=7,5
   - Parfum, conservateurs qs
   - Eau qsp 100 g

### EXEMPLE 2

On prépare un shampooing de composition suivante :
- Tensio-actif de type acide éther carboxylique polyoxyéthyléné, de formule : R(OCH₂CH₂)ₙ OCH₂COOH
   dans laquelle :
   R = octylphényl
   n = valeur moyenne de 4
   vendu sous la dénomination AKYPO OP 40
   par la Société CHEM Y à 90% de MA 8,0 g MA
- Laurylsulfate d'ammonium à 30% de MA 6,0 g MA
- α-oléfinesulfonate de sodium à 38% de MA 6,0 g MA
- Polydiméthylsiloxane vendu sous la dénomination SILBIONE HUILE 47 V 500.000 par la Société RHONE POULENC 2,0 g
- Copolymère de chlorure de diallyl diméthyl ammonium et d'acide acrylique, vendu sous la dénomination MERQUAT 280 par la Société CALGON à 35% de MA 0,3 g MA
- Chlorure de sodium 4,0 g
- Hydroxyde de sodium qs pH=4,4
- Parfum, conservateurs qs
- Eau qsp 100 g

### EXEMPLE 3

On prépare un shampooing de composition suivante :
- Tensio-actif de type acide éther carboxylique polyoxyéthyléné, de formule :

   R(OCH₂CH₂)ₙ OCH₂COOH

   dans laquelle :
   R = alkyl(C₁₂-C₁₄)
   n = valeur moyenne de 2,5
   vendu sous la dénomination AKYPO RLM
   25 par la Société CHEM Y à 90% de MA 5,0 g MA
- Laurylsulfate de triéthanolamine à 40% de MA 10,0 g MA
- Cocoylbétaïne à 32% de MA 2,0 g MA
- Copolymère de chlorure de diallyl diméthyl ammonium et d'acide acrylique, vendu sous la dénomination MERQUAT 280 par la Société CALGON à 35% de MA 2,0 g MA
- Mélange de deux PDMS de viscosités différentes, vendu sous la dénomination CF 1241 par la Société GENERAL ELECTRIC 3,0 g
- Scléroglucane vendu à 90% de MA sous la dénomination ACTIGUM CS 11 par la Société SANOFI BIO INDUSTRIE 0,9 g MA
- Chlorure de sodium 3,0 g
- Triéthanolamine qs pH=6
- Parfum, conservateurs qs
- Eau qsp 100 g

### EXEMPLE 4

On prépare un shampooing de composition suivante :

| | |
|---|---|
| - Lauryléthersultate de sodium oxyéthyléné à 2 moles d'oxyde d'éthylène à 28% de MA | 12,0 g MA |
| - Cocoylbétaine à 32% de MA | 1,5 g MA |
| - Distéarate d'éthylèneglycol | 2,0 g |
| - Diéthanolamide d'acide de coprah | 1,5 g |
| - Copolymère de chlorure de diallyl diméthyl ammonium et d'acide acrylique, vendu sous la dénomination MERQUAT 280 par la Société CALGON à 35% de MA | 0,5 g MA |
| - Mélange de deux PDMS de viscosités différentes, vendu sous la dénomination CF 1241 par la Société GENERAL ELECTRIC | 2,5 g |
| - Triéthanolamine qs pH=6,8 | |
| - Parfum, conservateurs qs | |
| - Eau | qsp 100 g |

### EXEMPLE 5

On prépare un shampooing de composition suivante :

| | |
|---|---|
| - Laurylsuliate de sodium à 85% de MA | 30,0 g MA |
| - Lauroylsarcosinate de sodium à 30% de MA | 4,0 g MA |
| - Polydiméthylsiloxane vendu sous la dénomination SILBIONE HUILE 47V 500.000 par la Société RHONE POULENC | 3,0 g |
| - Copolymère de chlorure de diallyl diméthyl ammonium et d'acide acrylique, vendu sous la dénomination MERQUAT 280 par la Société CALGON à 35% de MA | 2,0 g MA |
| - Diéthanolamide d'acide laurique | 2,0 g |
| - Monoéthanolamide d'acide de coprah | 2,0 g |
| - PEG 150 distéarate | 0,4 g |
| - Séquestrant | 0,2 g |
| - Acide citrique qs pH=6,5 | |
| - Eau | qsp 100 g |

### EXEMPLE 6

On prépare un shampooing de composition suivante :
- Tensio-actif de type acide éther carboxylique polyoxyéthyléné, de formule :

   R(OCH₂CH₂)ₙOCH₂COO)H

   dans laquelle :
   R = nonylphénol
   n = valeur moyenne de 7 vendu sous la dénomination AKYPO
   NP 70 par la Société CHEM Y à
   90% de MA 10,0 g MA
- Laurylsulfate de triéthanolamine à 40% de MA 10,0 g MA
- Copolymère de chlorure de diallyl diméthyl ammonium et d'acide acrylique, vendu sous la dénomination MERQUAT 280 par la Société CALGON à 35% de MA 1,0 g MA
- Polyorganosiloxane à groupements hydroxyacylamino vendu sous la dénomination Q2 8413 par la Société DOW CORNING 2,5 g
- Diéthanolamide d'acide de coprah 2,5 g
- Chlorure de sodium 3,0 g
- Triéthanolamine qs pH=7,5
- Parfum, conservateurs qs
- Eau qsp 100,0 g

### EXEMPLE 7

On prépare un gel-douche de composition suivante :

| | |
|---|---|
| - Lauryl(C₁₂-C₁₄/70-30)éthersulfate de sodium oxyéthyléné à 2,2 moles d'oxyde d'éthylène, vendu à 28% de MA | 22,4 g MA |
| - Mélange de cocoylamidopropylbétaine et de monolaurate de glycérol (25/5) en solution aqueuse à 30% de MA, vendu sous la dénomination TEGOBETAINE HS par la Société GOLDSCHMIDT | 2.58 g MA |
| - Polydiméthylsiloxane (PM 250.000) vendu sous la dénomination SiLBIONE 70047 V 500.000 par la Société RHONE POULENC | 2,0 g |
| - Copolymère de chlorure de diallyl diméthyl ammonium et d'acide acrylique, vendu sous la dénomination MERQUAT 280 par la Société CALGON à 35% de MA | 0,5 g MA |
| - Distéarate d'éthylèneglycoi(C₁₆-C₁₈/70-30) | 2,0 g |
| - Triéthanolamine qs pH = 7 | |
| - Parfum, conservateurs qs | |
| - Eau | qsp 100.0 g |

## Revendications (Revendications pour l'(les) Etat(s) contractant(s) suivant(s): AT, BE, CH, DE, FR, GB, GR, IT, LI, NL, PT)

1. Composition de lavage et/ou de conditionnement des matières kératiniques, **caractérisée par le fait qu'**elle contient dans un milieu aqueux, un agent tensio-actif possédant des propriétés détergentes, au moins une silicone insoluble dans le milieu et au moins un copolymère dérivé de diallyldialkylamrnonium et d'un monomère anionique; et ne contenant pas d'agents de mise en suspension constitués par des alcools ayant 27 à 44 atomes de carbone et comportant un ou deux groupements éther et/ou thioéthers ou sulfoxyde; l'agent tensio-actif possédant des propriétés détergentes étant présent dans les compositions dans des proportions comprises entre 5 et 50% en poids par rapport au poids total de la composition.

2. Composition selon la revendication 1, **caractérisée par le fait que** la silicone est choisie parmi les polyorganosiloxanes insolubles dans le milieu aqueux et se présente sous forme d'huile, de cire, de gomme ou de résine.

3. Composition selon la revendication 2, **caractérisée par le fait que** les polyorganosiloxanes sont choisis parmi les silicones volatiles.

4. Composition selon la revendication 3, **caractérisée par le fait que** les silicones volatiles sont choisies parmi :
- les silicones cycliques comportant de 3 à 7 atomes de silicium:
- les cyclopolymères du type diméthylsiloxané/méthylalkylsiloxane;
- les mélanges de silicones cycliques avec des composés organiques dérivés du silicium;
- les silicones volatiles linéaires ayant 2 à 9 atomes de silicium et de viscosité inférieure ou égale à 5.10⁻⁶m²/s à 25°C.

5. Composition selon la revendication 2, **caractérisée par le fait que** les polyorganosiloxanes sont choisis parmi les silicones non volatiles comprenant les polyalkylsiloxanes, les polyalkylarylsiloxanes, les polyarylsiloxanes, les gommes et résines de silicone, les polysiloxanes organomodifiés et leur(s) mélange(s).

6. Composition selon la revendication 5, **caractérisée par le fait que** les polyorganosiloxanes non volatils sont choisis parmi :
(a) les polyalkylsiloxanes, choisis parmi :
- les polydiméthylsiloxanes linéaires à groupements terminaux triméthylsilyle, de viscosité comprise entre 5.10⁻⁶ et 2,5 m²/s à 25°C;
- les polydiméthylsiloxanes linéaires à groupements terminaux diméthylsilanol;
- les polyalkyl(C₁-C₂₀)siloxanes;
(b) les polyalkylarylsiloxanes, choisis parmi :
- les polydiméthylméthyIphénylsiIoxanes, les polyméthylphénylsiloxanes, les polydiméthyldiphénylsiloxanes linéaires et/ou ramifiés, de viscosité comprise entre 1.10⁻⁵ et 5.10⁻² m²/s à 25°C;
(c) les gommes de silicone, choisies parmi les polydiorganosiloxanes ayant des masses moléculaires comprises entre 200 000 et 1 000 000 utilisées seules ou sous forme de mélange dans un solvant;
(d) les résines de silicones, constituées d'unités :
R'₂ Si O_{2/2}, R'Si O_{3/2}, Si O_{4/2}
dans lesquelles R' représente un groupement hydrocarboné ayant de 1 à 6 atomes de carbone ou un groupement phényle;
(e) les silicones organomodifiées, choisies parmi les silicones comportant dans leur structure un ou plusieurs groupements organo-fonctionnels directement fixés sur la chaîne siloxanique ou fixés par l'intermédiaire d'un radical hydrocarboné.

7. Composition selon la revendication 6, **caractérisée par le fait que** les gommes de silicone utilisées seules ou sous forme de mélange sont choisies parmi les silicones de structures suivantes :
- poly[(diméthylsiloxane)/(méthylvinylsiloxane)],
- poly[(diméthylsiloxane)/(diphénylsiloxane)],
- poly[(diméthylsiIoxanc)/(phénylméthyIsiloxane)],
- poly[(diméthylsiloxane)/(diphénylsiloxane)/méthylvinylsiloxane)]
et les mélanges suivants :
- des mélanges formés à partir d'un polydiméthylsiloxane hydroxylé en bout de chaîne et d'un polydiméthylsiloxane cyclique;
- les mélanges formés à partir d'une gomme polydiméthylsiloxane et d'une silicone cyclique; et
- des mélanges de polydiméthylsiloxanes de viscosités différentes.

8. Composition selon la revendication 6, **caractérisée par le fait que** les silicones organomodifiées sont choisies parmi les polyorganosiloxanes comportant :
a) des groupements polyéthylèneoxy et/ou polypropylèneoxy;
b) des groupements aminés substitués ou non;
c) des groupements thiols;
d) des groupements carboxylates;
e) des groupements alcoxylés;
f) des groupements hydroxyalkyle;
g) des groupements acyloxyalkyle;
h) des groupements alkylcarboxyliques;
i) des groupements 2-hydroxyalkylsulfonates;
j) des groupements 2-hydroxyalkylthiosulfates;
k) des groupements hydroxyacylamino.

9. Composition selon l'une quelconque des revendications 2 à 8, **caractérisée par le fait que** les polyorganosiloxanes sont choisis parmi les polyalkylsiloxanes linéaires à groupements terminaux triméthyl silyle, de viscosité comprise entre 0,2 et 2,5 m2/s à 25°C, les mélanges d'un polydiméthylsiloxane hydroxylé en bout de chaîne et d'un polydiméthylsiloxane cyclique, les mélanges de deux PDMS constitués d'une gomme et d'une huile de viscosités différentes, un polyorganosiloxane modifié par des groupements hydroxyacylarnino.

10. Composition selon l'une quelconque des revendications 1 à 9, **caractérisée par le fait que** le polymère dérivé de diallyldialkylammonium et d'un monomère anionique est constitué par un polymère comportant 60 à 99% en poids dudit polymère d'unités dérivées d'un monomère de diallyldialkylammonium quaternaire dans lequel les groupements alkyle ont, indépendamment l'un de l'autre, 1 à 18 atomes de carbone, l'anion correspondant étant dérivé d'un acide ayant une constante d'ionisation supérieure à 10⁻¹³ et environ 1 à 40% en poids dudit polymère étant constitué d'unités d'un monomère anionique choisi parmi l'acide acrylique et l'acide méthacrylique.

11. Composition selon la revendication 10, **caractérisée par le fait que** le poids moléculaire dudit polymère est compris entre 50.000 et 10.000.000 déterminé par chromatographie par perméation de gel.

12. Composition selon la revendication 10 ou 11, **caractérisée par le fait que** les groupements alkyle ont entre 1 et 4 atomes de carbone.

13. Composition selon l'une quelconque des revendications 10 à 12, **caractérisée par le fait que** le polymère est choisi parmi les copolymères de chlorure de diméthyldiallylammonium ou de diéthyldiallylammonium et d'acide acrylique.

14. Composition selon l'une quelconque des revendications 1 à 13, **caractérisée par le fait que** les agents tensio-actifs sont choisis parmi les agents tensio-actifs anioniques, amphotères, zwittérioniques, non-ioniques ou leurs mélanges, ayant des propriétés détergentes.

15. Composition selon l'une quelconque des revendications 1 à 14, **caractérisée par le fait que** l'agent tensio-actif à propriétés détergentes est présent dans les compositions dans des proportions comprises entre 8 et 35% en poids par rapport au poids total de la composition.

16. Composition selon l'une quelconque des revendications 1 à 15, **caractérisée par le fait que** la silicone est présente dans des proportions comprises entre 0,2 et 30% en poids, et de préférence entre 0,4 et 15% en poids par rapport au poids total de la composition.

17. Composition selon l'une quelconque des revendications 1 à 16, **caractérisée par le fait que** le polymère de diallyldialkylammonium et d'un monomère anionique, est présent dans des proportions comprises entre 0,1 et 10% en poids par rapport au poids total de la composition, et de préférence entre 0,5 et 5% en poids par rapport au poids total de la composition.

18. Composition selon l'une quelconque des revendications 1 à 17, **caractérisée par le fait que** le milieu aqueux est constitué par de l'eau ou par un mélange d'eau et d'un solvant cosmétiquement acceptable.

19. Composition selon l'une quelconque des revendications 1 à 18, **caractérisée par le fait que** la composition contient également un agent de mise en suspension autre qu'un alcool ayant 27 à 44 atomes de carbone et comportant un à deux groupements éther et/ou thioéther ou sulfoxyde.

20. Composition selon l'une quelconque des revendications 1 à 19, **caractérisée par le fait que** l'agent de suspension est choisi parmi :
a)
RX (III)
dans laquelle R est un radical aliphatique à longue chaîne carbonée, éventuellement interrompue par des atomes d'oxygène, et X est un reste acide carboxylique, sulfurique ou phosphorique ou un radical dérivé d'un acide carboxylique ou d'un amide; ces composés de formule (III) sont choisis parmi ceux dans lesquels :
(i) R est un radical alkyle ou alcényle en C₁₁-C₂₁
X est :
· un groupement COOA où A est un radical mono ou polyhydroxy alkyle dérivé d'un polyol en C₂-C₃ ou un radical CH₂CH₂SO₃M,
· un groupement CO(OCH₂CH₂)ₙ-OH où n a une valeur comprise entre 2 et 150,
· un groupement 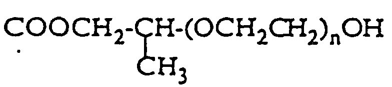 où n a une valeur comprise entre 2 et 150, les fonctions OH libres des groupements définis ci-dessus pouvant être estérifiées par un acide RCOOH où R est un alkyle ou un alcényle en C₁₁-C₂₁,
· un groupement CONR₁R₂ où R₁ et R₂ représentent hydrogène ou: hydroxyalkyle en C₁-C₄, l'un au moins représentant hydroxyalkyle en C₁-C₄,
· un groupement OSO₃M ou 1/3 PO₄³⊖M₃ où M représente un métal alcalin, ammonium ou un reste d'alcanolamine en C₁-C₄;
(ii) R désigne un radical R₃(OC₂H₄)ℓ OCH₂ et X désigne un groupement COOM où M a la signification indiquée ci-dessus, R₃ désignant un radical alkyle en C₁₂-C₁₄ et ℓ un nombre entier ou décimal compris entre 2,5 et 10, ou bien R₃ désigne oléyle, et ℓ varie de 2 à 9 ou encore R₃ désigne alkyl(C₈-C₉)phényle et ℓ varie de 4 à 8, ou les dérivés dans lesquels R désigne un groupement alkyl(C₁₂-C₁₆) éther et X un groupement CONR₁R₂, dans lequel R₁ et R₂ ont la même signification que celle indiquée ci-dessus;
b) des oxydes de diméthylalkyl(C₁₆-C₂₂)amines;
c) les biopolysaccharides.

21. Composition selon l'une des revendications 19 ou 20, **caractérisée par le fait que** l'agent de mise en suspension est présent dans des proportions comprises entre 0,1 et 20% en poids du poids total de la composition.

22. Composition selon l'une quelconque des revendications 1 à 21, **caractérisée par le fait qu'**elle contient en outre un adjuvant choisi parmi les agents nacrants ou opacifiants présents dans des proportions allant jusqu'à 3% du poids total de la composition, des agents régulateurs de viscosité choisis parmi les électrolytes, des hydrotropes et les agents épaississants présents dans des proportions allant jusqu_{'}à 10% en poids par rapport au poids total de la composition.

23. Composition selon l'une quelconque-des revendications 1 à 22, **caractérisée par le fait qu'**elle contient en outre des agents ayant pour effet d'améliorer les propriétés cosmétiques des cheveux et/ou de la peau, choisis parmi les agents tensio-actifs cationiques, les polymères autres que les copolymères de diallyldiallcylammonium et d'un monomère anionique, des protéines ou des silicones solubles dans le milieu.

24. Procédé de lavage et/ou de conditionnement des matières kératiniques, **caractérisé par le fait que** l'on applique sur ces matières au moins une composition telle que définie dans l'une quelconque des revendications 1 à 23, et qu'on procède ensuite à un rinçage.

25. Utilisation pour le lavage des cheveux d'une composition telle que définie dans l'une quelconque des revendications 1 à 23.

26. Utilisation pour le lavage de la peau d'une composition telle que définie dans l'une quelconque des revendications 1 à 23.

## Revendications (Revendications pour l'(les) Etat(s) contractant(s) suivant(s): ES)

1. Composition de lavage et/ou de conditionnement des matiè res kératiniques, **caractérisée par le fait qu'**elle contient dans un milieu aqueux, un agent tensio-actif possédant des propriétés détergentes, au moins une silicone insoluble dans le milieu et au moins un copolymère dérivé de diallyldialkylammonium et d'un monomère anionique; et ne contenant pas d'agents de mise en suspension constitués par des alcools ayant 27 à 44 atomes de carbone et comportant un ou deux groupements éther et/ou thioéthers ou sulfoxyde; l'agent tensio-actif possédant des propriétés détergentes étant présent dans les compositions dans des proportions comprises entre 5 et 50% en poids par rapport au poids total de la composition.

2. Composition selon la revendication 1, **caractérisée par le fait que** la silicone est choisie parmi les polyorganosiloxanes insolubles dans le milieu aqueux' et se présente sous forme d'huile, de cire, de gomme ou de résine.

3. Composition selon la revendication 2, **caractérisée par le fait que** les polyorganosiloxanes sont choisis parmi les silicones volatiles.

4. Composition selon la revendication 3, **caractérisée par le fait que** les silicones volatiles sont choisies parmi :
- les silicones cycliques comportant de 3 à 7 atomes de silicium;
- les cyclopolymères du type diméthylsiloxane/méthylalkylsiloxane;
- les mélanges de silicones cycliques avec des composés organiques-dérivés du silicium;
- les silicones volatiles linéaires ayant 2 à 9 atomes de silicium et de viscosité inférieure ou égale à 5.10⁻⁶m²/s à 25°C.

5. Composition selon la revendication 2, **caractérisée par le fait que** les polyorganosiloxanes sont choisis parmi les silicones non volatiles comprenant les polyalkylsiloxanes, les polyalkylarylsiloxarics, les polyarylsiloxanes, les gommes et résines de silicone, les polysiloxanes organomodifiés et leur(s) mélange(s).

6. Composition selon la revendication 5, **caractérisée par le fait que** les polyorganosiloxanes non volatils sont choisis parmi :
(a) les polyalkylsiloxanes, choisis parmi :
- les polydiméthylsiloxanes linéaires à groupements terminaux triméthylsilyle, de viscosité comprise entre 5.10⁻⁶ et 2,5 m²/s à 25°C;
- les polydiméthylsiloxanes linéaires à groupements terminaux diméthylsilanol;
- les polyalkyl(C₁-C₂₀)siloxanes;
(b) les polyalkylarylsiloxanes, choisis parmi :
- les polydiméthylméthylphénylsiloxanes, les polyméthylphénylsiloxanes, les polydiméthyldiphénylsiloxanes linéaires et/ou ramifiés, de viscosité comprise entre 1.10⁻⁵ et 5.10⁻² m²/s à 25°C;
(c) les gommes de silicone, choisies parmi les polydiorganosiloxanes ayant des masses moléculaires comprises entre 200 000 et 1 000 000 utilisées seules ou sous forme de mélange dans un solvant;
(d) les résines de silicones, constituées d'unités :
R'₂ Si O_{2/2}, R'Si O_{3/2}, Si O_{4/2}
dans lesquelles R' représente un groupement hydrocarboné ayant de 1 à 6 atomes de carbone ou un groupement phényle;
(e) les silicones organomodifiées, choisies parmi les silicones comportant dans leur structure un ou plusieurs groupements organo-fonctionnels directement fixés sur la chaîne siloxanique ou fixés par l'intermédiaire d'un radical hydrocarboné.

7. Composition selon la revendicàtion 6, **caractérisée par le fait que** les gommes de silicone utilisées seules ou sous forme de mélange sont choisies parmi les silicones de structures suivantes :
- poly[(diméthylsiloxane)/(méthylvinylsiloxane)],
- poly[(diméthylsiloxane)/(diphénylsiloxane)],
- poly[(diméthylsiloxane)/(phénylméthylsiloxane)],
- poly[(diméthylsiloxane)/(diphénylsiloxane)/méthylvinylsiloxane)]
et les mélanges suivants :
- des mélanges formés à partir d'un polydiméchylsiloxane hydroxylé en bout de chaîne et d'un polydiméthylsiloxane cyclique;
- les mélanges formés à partir d'une gomme polydiméthylsiloxane et d'une silicone cyclique; et
- des mélanges de polydiméthylsiloxanes de viscosités différentes.

8. Composition selon la revendication 6, **caractérisée par le fait que** les silicones organomodifiées sont choisies parmi les polyorganosiloxanes comportant :
a) des groupements polyéthylèneoxy et/ou polypropylèneoxy;
b) des groupements aminés substitués ou non;
c) des groupements thiols;
d) des groupements carboxylates;
e) des groupements alcoxylés;
f) des groupements hydroxyalkyle;
des groupements acyloxyalkyle;
h) des groupements alkylcarboxyliques;
i) des groupements 2-hydroxyalkylsulfonates;
j) des groupements 2-hydroxyalkylthiosulfates;
k) des groupements hydroxyacylamino.

9. Composition selon l'une quelconque des revendications 2 à 8, **caractérisée par le fait que** les polyorganosiloxanes sont choisis parmi les polyalkylsiloxanes linéaires à groupements terminaux triméthyl silyle, de viscosité comprise entre 0,2 et 2,5 m2/s à 25°C, les mélanges d'un polydiméthylsiloxane hydroxylé en bout de chaîne et d'un polydiméthylsiloxane cyclique, les mélanges de deux PDMS constirués d'une gomme et d'une huile de viscosités différentes, un polyorganosiloxane modifié par des groupements hydroxyacylamino.

10. Composition selon l'une quelconque des revendications 1 à 9, **caractérisée par le fait que** le polymère dérivé de diallyldialkylammonium et d'un monomère anionique est constitué par un polymère comportant 60 à 99% en poids dudit polymère d'unités dérivées d'un monomère de diallyldialkylammonium quaternaire dans lequel les groupements alkyle ont, indépendamment l'un de l'autre, 1 à 18 atomes de carbone, l'anion correspondant étant dérivé d'un acide ayant une constante d'ionisation supérieure à 10⁻¹³ et environ 1 à 40% en poids dudit polymère étant constitué d'unités d'un monomère anionique choisi parmi l'acide acrylique et l'acide méthacrylique.

11. Composition selon la revendication 10, **caractérisée par le fait que** le poids moléculaire dudit polymère est compris entre 50.000 et 10.000.000 déterminé par chromatographie par perméation de gel.

12. Composition selon la revendication 10 ou 11, **caractérisée par le fait que** les groupements alkyle ont entre 1 et 4 atomes de carbone.

13. Composition selon l'une quelconque des revendications 10 à 12, **caractérisée par le fait que** le polymère est choisi parmi les copolymères de chlorure de diméthyldiallylammonium ou de diéthyldiallylammonium et d'acide acrylique.

14. Composition selon l'une quelconque des revendications 1 à 13, **caractérisée par le fait que** les agents tensio-actifs sont choisis parmi les agents tensio-actifs anioniques, amphotères, zwittérioniques, non-ioniques ou leurs mélanges, ayant des propriétés détergentes.

15. Composition selon l'une quelconque des revendications 1 à 14, **caractérisée par le fait que** l'agent tensio-actif à propriétés détergentes est présent dans les compositions dans des proportions comprises entre 8 et 35% en poids par rapport au poids total de la composition.

16. Composition selon l'une quelconque des revendications 1 à 15, **caractérisée par le fait que** la silicone est présente dans des proportions comprises entre 0,2 et 30% en poids, et de préférence entre 0,4 et 15% en poids par rapport au poids total de la composition.

17. Composition selon l'une quelconque des revendications 1 à 16, **caractérisée par le fait que** le polymère de diallyldialkylammonium et d'un monomère anionique, est présent dans des proportions comprises entre 0,1 et 10% en poids par rapport au poids total de la composition, et de préférence entre 0,5 et 5% en poids par rapport au poids total de la composition.

18. Composition selon l'une quelconque des revendications 1 à 17, **caractérisée par le fait que** le milieu aqueux est constitué par de l'eau ou par un mélange d'eau et d'un solvant cosmétiquement acceptable.

19. Composition selon l'une quelconque des revendications 1 à 18, **caractérisée par le fait que** la composition contient également un agent de mise en suspension autre qu'un alcool ayant 27 à 44 atomes de carbone et comportant un à deux groupements éther et/ou thioéther ou sulfoxyde.

20. Composition selon l'une quelconque des revendications 1 à 19, **caractérisée par le fait que** l'agent de suspension est choisi parmi :
a)
RX (III)
dans laquelle R est un radical aliphatique à longue chaîne carbonée, éventuellement interrompue par des atomes d'oxygène, et X est un reste acide carboxylique, sulfurique ou phosphorique ou un radical dérivé d'un acide carboxylique ou d'un amide; ces composés de formule (III) sont choisis parmi ceux dans lesquels :
Ci) R est un radical alkyle ou alcényle en C₁₁-C₂₁
X est :
· un groupement COOA où A est un radical mono ou polyhydroxy alkyle dérivé d'un polyol en C₂-C₃ ou un radical CH₂CH₂SO₃M,
· un groupement CO(OCH₂CH₂)ₙ-OH où n a une valeur comprise entre 2 et 150,
· un groupement 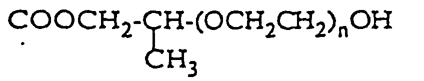 où n a une valeur comprise entre 2 et 150, les fonctions OH libres des groupements définis ci-dessus pouvant être estérifiées par un acide RCOOH où R est un alkyle ou un alcén^{y}le en C₁₁-C₂₁,
· un groupement CONR₁R₂ où R₁ et R₂ représentent hydrogène ou: hydroxyalkyle en C₁-C₄, l'un au moins représentant hydroxyalkyle en C₁-C₄,
· un groupement OSO₃M ou 1/3 PO₄³ ⊖M₃ où M représente un métal alcalin, ammonium ou un reste d'alcanolamine en C₁-C₄;
(ii) R désigne un radical R₃(OC₂H₄)ℓ OCH₂ et X désigne un groupement COOM où M a la signification indiquée ci-dessus, R³ désignant un radical alkyle en C₁₂-C₁₄ et ℓ un nombre entier ou décimal compris entre 2,5 et 10, ou bien R₃ désigne oléyle et ℓ varie de 2 à 9 ou encore R₃ désigne alkyl(C₈-C₉)phényle et ℓ varie de 4 à 8, ou les dérivés dans lesquels R désigne un groupement alkyl(C₁₂-C₁₆) éther et X un groupement CONR₁R₂, dans lequel R₁ et R₂ ont la même signification que celle indiquée ci-dessus;
b) des oxydes de diméthylalkyl(C₁₆-C₂₂)amines;
c) les biopolysaccharides.

21. Composition selon l'une des revendications 19 ou 20, **caractérisée par le fait que** l'agent de mise en suspension est présent dans des proportions comprises entre 0,1 et 20% en poids du poids total de la composition.

22. Composition selon l'une quelconque des revendications 1 à 21, **caractérisée par le fait qu'**elle contient en outre un adjuvant choisi parmi les agents nacrants ou opacifiants présents dans des proportions allant jusqu'à 3% du poids total de la composition, des agents régulateurs de viscosité choisis parmi les électrolytes, des hydrotropes et les agents épaississants présents dans des proportions allant jusqu'à 10% en poids par rapport au poids total de la composition.

23. Composition selon l'une quelconque des revendications 1 à 22, **caractérisée par le fait qu'**elle contient en outre des agents ayant pour effet d'améliorer les propriétés cosmétiques des cheveux et/ou de la peau, choisis parmi les agents tensio-actifs cationiques, les polymères autres que les copolymères de diallyldialkylammonium et d'un monomère anionique, des protéines ou des silicones solubles dans le milieu.

24. Procédé de lavage et/ou de conditionnement des matières kératiniques, **caractérisé par le fait que** l'on applique sur ces matières au moins une composition telle que définie dans l'une quelconque des revendications 1 à 23, et qu'on procède ensuite à un rinçage.

25. Utilisation pour le lavage des cheveux d'une composition telle que définie dans l'une quelconque des revendications 1 à 23.

26. Utilisation pour le lavage de la peau d'une composition telle que définie dans l'une quelconque des revendications 1 à 23.

27. Procédé de préparation d'une composition de lavage et/ou de conditionnement des matières kératiniques, **caractérisé par le fait que** l'on introduit dans un milieu aqueux constitué par de l'eau ou un mélange d'eau et d'un solvant cosmétiquement acceptable:
a) au moins un agent tensio-actif choisi parmi les agents tensio-actifs anioniques, amphotères, zwittérioniques, non-ioniques ou leurs mélanges, dans des proportions suffisantes pour conférer à la composition un caractère détergent, comprises entre 5 et 50% en poids et en particulier entre 8 et 35% en poids par rapport au poids total de la composition;
b) au moins une silicone insoluble dans le milieu aqueux utilisé, dans des proportions comprises entre 0,2 et 30% et de préférence entre 0,4 et 15% en poids par rapport au poids total de la composition; et
c) au moins un copolymère dérivé de diallyldiallcylammonium et d'un monomère anionique dans des proportions comprises entre 0,1 et 10% et de préférence entre 0,5 et 5% en poids par rapport au poids total de la composition; et que l'on ajoute optionnellement:
d) entre 0,1 et 20% en poids par rapport au poids total de la composition d'un agent de mise en suspension autre qu'un alcool en C₂₇-C₄₄ et comportant un ou deux groupements éther et/ou thioéther ou sulfoxyde;
e) jusqu'à 3% du poids total de la composition d'agents nacrants ou opacifiants;
f) jusqu'à 10% du poids total de la composition d'agents régulateurs de viscosité;
g) d'autres adjuvants choisis parmi les polymères autres que les copolymères de diallyldialkylammonium et d'un monomère anionique, les tensio-actifs cationiques, les protéines, les silicones solubles dans le milieu, les parfums, conservateurs, séquestrants, stabilisateurs de mousse, propulseurs, colorants, filtres solaires et agents acidifiants ou alcalinisants.

## Patentansprüche (Patentansprüche für folgende(n) Vertragsstaat(en): AT, BE, CH, DE, DK, FR, GB, GR, IT, LI, NL, PT, SE)

1. Zusammensetzung zum Waschen und/oder Konditionieren keratinischer Materialien,
**dadurch gekennzeichnet, daß**
sie in einem wässrigen Milieu ein oberflächenaktives Mittel mit reinigenden Eigenschaften, mindestens ein in dem Milieu unlösliches Silicon und mindestens ein Copolymer enthält, das von einer Diallyldialkylammoniumverbindung und einem anionischen Monomer abgeleitet ist, wobei keine Suspendiermittel aus Alkoholen mit 27 bis 44 Kohlenstoffatomen und einer oder zwei Ether- und/oder Thioetheroder Sulfoxid-Gruppierungen enthalten sind und das oberflächenaktive Mittel mit den reinigenden Eigenschaften in den Zusammensetzungen mit Mengenanteilen von 5 bis 50 Gew.-% vorhanden ist, bezogen auf das Gesamtgewicht der Zusammensetzung.

2. Zusammensetzung gemäß Anspruch 1,
**dadurch gekennzeichnet, daß**
das Silicon aus in dem wässrigen Milieu unlöslichen Polyorganosiloxanen ausgewählt ist und in Form eines Öls, Wachses, Gummi- oder Harzprodukts vorliegt.

3. Zusammensetzung gemäß Anspruch 2,
**dadurch gekennzeichnet, daß**
die Polyorganosiloxane aus flüchtigen Siliconen ausgewählt sind.

4. Zusammensetzung gemäß Anspruch 3,
**dadurch gekennzeichnet, daß**
die flüchtigen Silicone ausgewählt sind aus:
- cyclischen Siliconen mit 3 bis 7 Siliciumatomen;
- Cyclopolymeren des Typs Dimethylsiloxan/Methylalkylsiloxan;
- Mischungen aus cyclischen Siliconen mit organischen Siliciumderivatverbindungen;
- flüchtigen linearen Siliconen mit 2 bis 9 Siliciumatomen und einer Viskosität bei 25°C von weniger als oder gleich 5 x 10⁻⁶ m²/s.

5. Zusammensetzung gemäß Anspruch 2,
**dadurch gekennzeichnet, daß**
die Polyorganosiloxane aus nicht-flüchtigen Siliconen ausgewählt sind, die Polyalkylsiloxane, Polyalkylarylsiloxane, Polyarylsiloxane, Gummi- und Harzprodukte aus Silicon, organomodifizierte Polysiloxane und deren Mischung(en) umfassen.

6. Zusammensetzung gemäß Anspruch 5,
**dadurch gekennzeichnet, daß**
die nicht-flüchtigen Polyorganosiloxane ausgewählt sind aus:
(a) Polyalkylsiloxanen, ausgewählt aus:
- linearen Polydimethylsiloxanen mit endständigen Trimethylsilyl-Gruppierungen einer Viskosität bei 25°C von 5 x 10⁻⁶ bis 2,5 m²/s;
- linearen Polydimethylsiloxanen mit endständigen Dimethylsilanol-Gruppierungen;
- Poly-C₁₋₂₀-alkylsiloxanen;
(b) Polyalkylarylsiloxanen, ausgewählt aus:
- linearen und/oder verzweigten Polydimethylmethylphenylsiloxanen, Polymethylphenylsiloxanen und Polydimethyldiphenylsiloxanen einer Viskosität bei 25°C von 1 x 10⁻⁵ bis 5 x 10⁻² m²/s;
(c) Gummiprodukten aus Silicon, ausgewählt aus Polydiorganosiloxanen mit Molekularmassen von 200000 bis 1000000, die alleine oder in Form einer Mischung in einem Lösungsmittel verwendet werden;
(d) Harzprodukten aus Siliconen, aufgebaut aus Einheiten: R'₂SiO_{2/2}, R'SiO_{3/2}, SiO_{4/2},
in denen R' eine Kohlenwassserstoffgruppe mit 1 bis 6 Kohlenstoffatomen oder eine Phenylgruppe darstellt;
(e) organomodifizierten Siliconen, ausgewählt aus Siliconen, die in ihrer Struktur eine oder mehrere organofunktionelle Gruppierungen aufweisen, die direkt oder über das Zwischenglied eines Kohlenwasserstoffrests an die Siloxan-Kette gebunden sind.

7. Zusammensetzung gemäß Anspruch 6,
**dadurch gekennzeichnet, daß**
die Gummiprodukte aus Silicon, die alleine oder in Form einer Mischung verwendet werden, aus Siliconen folgender Strukturen:
- Poly((dimethylsiloxan)/(methylvinylsiloxan)),
- Poly((dimethylsiloxan)/(diphenylsiloxan)),
- Poly((dimethylsiloxan)/(phenylmethylsiloxan)),
- Poly((dimethylsiloxan)/(diphenylsiloxan)/(methylvinylsiloxan))
und aus folgenden Mischungen ausgewählt sind:
- Mischungen, die aus einem am Kettenende hydroxylierten Polydimethylsiloxan und einem cyclischen Polydimethylsiloxan gebildet sind;
- Mischungen, die aus einem Polydimethylsiloxan-Gummi und einem cyclischen Silicon gebildet sind; und
- Mischungen aus Polydimethylsiloxanen unterschiedlicher Viskositäten.

8. Zusammensetzung gemäß Anspruch 6,
**dadurch gekennzeichnet, daß**
die organomodifizierten Silicone aus Polyorganosiloxanen ausgewählt sind, die aufweisen:
a) Polyethylenoxy- und/oder Polypropylenoxy-Gruppierungen,
b) aminierte Gruppierungen, substituiert oder nicht,
c) Thiol-Gruppierungen,
d) Carboxylat-Gruppierungen,
e) alkoxylierte Gruppierungen,
f) Hydroxyalkyl-Gruppierungen,
g) Acyloxyalkyl-Gruppierungen,
h) Alkylcarboxyl-Gruppierungen,
i) 2-Hydroxyalkylsulfonat-Gruppierungen,
j) 2-Hydroxyakylthiosulfat-Gruppierungen und
k) Hydroxacylamino-Grupierungen.

9. Zusammensetzung gemäß jedem der Ansprüche 2 bis 8,
**dadurch gekennzeichnet, daß**
die Polyorganosiloxane aus linearen Polyalkylsiloxanen mit endständigen Trimethylsilylgruppierungen einer Viskosität bei 25°C von 0,2 bis 2,5 m²/s, aus Mischungen eines am Kettenende hydroxylierten Polydimethylsiloxans und einem cyclischen Polydimethylsiloxan, aus Mischungen aus 2 PDMS-Produkten unterschiedlicher Viskositäten aus einem Gummiprodukt und einem Öl sowie aus einem mit Hydroxyacylamino-Gruppierungen modifizierten Polyorganosiloxan ausgewählt sind.

10. Zusammensetzung gemäß jedem der Ansprüche 1 bis 9,
**dadurch gekennzeichnet, daß**
das aus einer Diallyldialkylammoniumverbindung und einem anionischen Monomer abgeleitete Polymer aus einem Polymer zusammengesetzt ist, das 60 bis 99 Gew.% des genannten Polymer an Einheiten aus einem Monomer einer quaternären Diallyldialkylammoniumverbindung enthält, in der die Alkylgruppen, unabhängig voneinander, 1 bis 18 Kohlenstoffatome aufweisen und das entsprechende Anion aus einer Säure mit einer Ionisierungskonstante von mehr als 10-13 stammt, wobei ca. 1 bis 40 Gew.% des genannten Polymer aus Einheiten eines anionischen Monomer zusammengesetzt sind, das aus Acryl- und Methacrylsäure ausgewählt ist.

11. Zusammensetzung gemäß Anspruch 10,
**dadurch gekennzeichnet, daß**
das Molekulargewicht des genannten Polymer 50000 bis 10000000 beträgt, bestimmt durch Gelpermeationschromatographie.

12. Zusammensetzung gemäß Anspruch 10 oder 11,
**dadurch gekennzeichnet, daß**
die Alkylgruppen 1 bis 4 Kohlenstoffatome aufweisen.

13. Zusammensetzung gemäß jedem der Ansprüche 10 bis 12,
**dadurch gekennzeichnet, daß**
das Polymer aus Copolymeren von Dimethyldiallylammonium- oder Diethyldiallylammoniumchlorid und Acrylsäure ausgewählt ist.

14. Zusammensetzung gemäß jedem der Ansprüche 1 bis 13,
**dadurch gekennzeichnet, daß**
die oberflächenaktiven Mittel, die reinigende Eigenschaften aufweisen, aus anionischen, amphoteren, zwitterionischen und nicht-ionischen oberflächenaktiven Mitteln oder aus deren Mischungen ausgewählt sind.

15. Zusammensetzung gemäß jedem der Ansprüche 1 bis 14,
**dadurch gekennzeichnet, daß**
das oberflächenaktive Mittel mit reinigenden Eigenschaften in den Zusammensetzungen in Mengenanteilen von 8 bis 35 Gew.% vorhanden ist, bezogen auf das Gesamtgewicht der Zusammensetzung.

16. Zusammensetzung gemäß jedem der Ansprüche 1 bis 15,
**dadurch gekennzeichnet, daß**
das Silicon in Mengenanteilen von 0,2 bis 30 und vorzugsweise von 0,4 bis 15 Gew.% vorhanden ist, bezogen auf das Gesamtgewicht der Zusammensetzung.

17. Zusammensetzung gemäß jedem der Ansprüche 1 bis 16,
**dadurch gekennzeichnet, daß**
das Polymer aus Diallyldialkylammoniumverbindung und anionischem Monomer in Mengenanteilen von 0,1 bis 10 und vorzugsweise von 0,5 bis 5 Gew.% vorhanden ist, bezogen auf das Gesamtgewicht der Zusammensetzung.

18. Zusammensetzung gemäß jedem der Ansprüche 1 bis 17,
**dadurch gekennzeichnet, daß**
das wässrige Milieu aus Wasser oder aus einer Mischung aus Wasser und einem kosmetisch geeigneten Lösungsmittel zusammengesetzt ist.

19. Zusammensetzung gemäß jedem der Ansprüche 1 bis 18,
**dadurch gekennzeichnet, daß**
die Zusammensetzung auch ein Mittel zur Suspendierung enthält, das sich von einem Alkohol unterscheidet, der 27 bis 44 Kohlenstoffatome aufweist und eine bis zwei Etherund/oder Thioether- oder Sulfoxid-Gruppierungen enthält.

20. Zusammensetzung gemäß jedem der Ansprüche 1 bis 19,
**dadurch gekennzeichnet, daß**
das Suspendiermittel ausgewählt ist aus:
a)
RX (III)
worin R ein aliphatischer Rest mit langer Kohlenstoffkette, die gegebenenfalls durch Sauerstoffatome unterbrochen ist, und X der Rest einer Carboxyl-, Schwefel- oder Phosphorsäure oder ein von einer Carboxylsäure oder einem Amid abgeleiteter Rest sind, wobei die Verbindungen der Formel (III) aus denjenigen ausgewählt sind, in denen gilt:
(i) R ist ein C₁₁₋₂₁-Alkyl- oder -Alkenylrest, X ist:
- eine Gruppierung COOA, worin A ein von einem C₂₋₃-Polyol abgeleiteter Mono- oder Polyhydroxyalkylrest oder ein Rest CH₂CH₂SO₃M ist,
- eine Gruppierung CO(OCH₂CH₂)ₙ-OH, worin n ein Wert von 2 bis 150 ist,
- eine Gruppierung COOCH₂-CH-CH₃-(OCH₂CH₂)ₙOH,
worin n ein Wert von 2 bis 150 ist, wobei die freien OH-Funktionen der oben definierten Gruppierungen mit einer Säure RCOOH verestert sein können, worin R ein C₁₁₋₂₁-Alkyl- oder -Alkenylrest ist,
- eine Gruppierung CONR₁R₂, worin R₁ und R₂ Wasserstoff oder einen Hydroxy-C₁₋₄-alkylrest darstellen, wobei mindestens einer einen Hydroxy-C₁₋₄-alkylrest darstellt,
- eine Gruppierung OSO₃M oder 1/3PO₄³⁻M₃, worin M ein Alkalimetall, einen Ammonium- oder C₁₋₄-Alkanolamin-Rest darstellt;
(ii) R bedeutet einen Rest R₃(OC₂H₄)ₗOCH₂, und X bedeutet eine Gruppierung COOM, worin M die oben angegebene Bedeutung hat, wobei R₃ einen C₁₂-₁₄-Alkylrest bedeutet und l eine ganze Zahl oder Dezimalzahl von 2,5 bis 10 ist, oder wobei R₃ auch einen Oleylrest bedeutet und l 2 bis 9 beträgt, oder wobei R₃ auch einen C₈₋₉-Alkylphenylrest bedeutet und l 4 bis 8 beträgt,
oder das Suspendiermittel ist aus Derivaten von RX ausgewählt, in denen R eine C₁₂₋₁₆-Alkylether-Gruppierung und X eine Gruppierung CONR₁R₂ bedeuten, in welcher R₁ und R₂ dieselbe Bedeutung haben, wie sie für die entsprechende Gruppierung oben angegeben ist;
b) Dimethyl-C₁₆₋₂₂-alkylaminoxiden;
c) Biopolysacchariden.

21. Zusammensetzung gemäß einem der Ansprüche 19 oder 20,
**dadurch gekennzeichnet, daß**
das Mittel zur Suspendierung in Mengenanteilen von 0,1 bis 20 Gew.% vorhanden ist, bezogen auf das Gesamtgewicht der Zusammensetzung.

22. Zusammensetzung gemäß jedem der Ansprüche 1 bis 21,
**dadurch gekennzeichnet, daß**
sie ausserdem einen Hilfsstoff, ausgewählt aus Perlmuttglanzmitteln oder opak machenden Mitteln, die in Mengenanteilen bis zu 3% des Gesamtgewichts der Zusammensetzung vorhanden sind, und Viskositätsreguliermittel enthält, die aus Elektrolyten, Hydrotropen und Verdickungsmitteln ausgewählt und in Mengenanteilen bis zu 10 Gew.% vorhanden sind, bezogen auf das Gesamtgewicht der Zusammensetzung.

23. Zusammensetzung gemäß jedem der Ansprüche 1 bis 22,
**dadurch gekennzeichnet, daß**
sie ausserdem Mittel, die die Wirkung aufweisen, die kosmetischen Eigenschaften der Haare und/oder der Haut zu verbessern, und aus kationischen oberflächenaktiven Mitteln und aus Polymeren ausgewählt sind, die sich von den Copolymeren einer Diallyldiammoniumverbindung und eines anionischen Monomer unterscheiden, sowie Proteine oder in dem Milieu lösliche Silicone enthält.

24. Verfahren zum Waschen und/oder Konditionieren keratinischer Materialien,
**dadurch gekennzeichnet, daß**
man auf diese Materialien mindestens eine in jedem der Ansprüche 1 bis 23 definierte Zusammensetzung aufbringt und sodann eine Spülung durchführt.

25. Verwendung einer in jedem der Ansprüche 1 bis 23 definierten Zusammensetzung zum Waschen der Haare.

26. Verwendung einer in jedem der Ansprüche 1 bis 23 definierten Zusammensetzung zum Waschen der Haut.

## Patentansprüche (Patentansprüche für folgende(n) Vertragsstaat(en): ES)

1. Zusammensetzung zum Waschen und/oder Konditionieren keratinischer Materialien,
**dadurch gekennzeichnet, daß**
sie in einem wässrigen Milieu ein oberflächenaktives Mittel mit reinigenden Eigenschaften, mindestens ein in dem Milieu unlösliches Silicon und mindestens ein Copolymer enthält, das von einer Diallyldialkylammoniumverbindung und einem anionischen Monomer abgeleitet ist, wobei keine Suspendiermittel aus Alkoholen mit 27 bis 44 Kohlenstoffatomen und einer oder zwei Ether- und/oder Thioetheroder Sulfoxid-Gruppierungen enthalten sind und das oberflächenaktive Mittel mit den reinigenden Eigenschaften in den Zusammensetzungen mit Mengenanteilen von 5 bis 50 Gew.-% vorhanden ist, bezogen auf das Gesamtgewicht der Zusammensetzung.

2. Zusammensetzung gemäß Anspruch 1,
**dadurch gekennzeichnet, daß**
das Silicon aus in dem wässrigen Milieu unlöslichen Polyorganosiloxanen ausgewählt ist und in Form eines Öls, Wachses, Gummi- oder Harzprodukts vorliegt.

3. Zusammensetzung gemäß Anspruch 2,
**dadurch gekennzeichnet, daß**
die Polyorganosiloxane aus flüchtigen Siliconen ausgewählt sind.

4. Zusammensetzung gemäß Anspruch 3,
**dadurch gekennzeichnet, daß**
die flüchtigen Silicone ausgewählt sind aus:
- cyclischen Siliconen mit 3 bis 7 Siliciumatomen;
- Cyclopolymeren des Typs Dimethylsiloxan/Methylalkylsiloxan;
- Mischungen aus cyclischen Siliconen mit organischen Siliciumderivatverbindungen;
- flüchtigen linearen Siliconen mit 2 bis 9 Siliciumatomen und einer Viskosität bei 25°C von weniger als oder gleich 5 x 10⁻⁶ m²/s.

5. Zusammensetzung gemäß Anspruch 2,
**dadurch gekennzeichnet, daß**
die Polyorganosiloxane aus nicht-flüchtigen Siliconen ausgewählt sind,d ie Polyalkylsiloxane, Polyalkylarylsiloxane, Polyarylsiloxane, Gummi- und Harzprodukte aus Silicon, organomodifizierte Polysiloxane und deren Mischung(en) umfassen.

6. Zusammensetzung gemäß Anspruch 5,
**dadurch gekennzeichnet, daß**
die nicht-flüchtigen Polyorganosiloxane ausgewählt sind aus:
(a) Polyalkylsiloxanen, ausgewählt aus:
- linearen Polydimethylsiloxanen mit endständigen Trimethylsilyl-Gruppierungen einer Viskosität bei 25°C von 5 x 10⁻⁶ bis 2,5 m²/s;
- linearen Polydimethylsiloxanen mit endständigen Dimethylsilanol-Gruppierungen;
- Poly-C₁₋₂₀-alkylsiloxanen;
(b) Polyalkylarylsiloxanen, ausgewählt aus:
- linearen und/oder verzweigten Polydimethylmethylphenylsiloxanen, Polymethylphenylsiloxanen und Polydimethyldiphenylsiloxanen einer Viskosität bei 25°C von 1 x 10⁻⁵ bis 5 x 10⁻² m²/s;
(c) Gummiprodukten aus Silicon, ausgewählt aus Polydiorganosiloxanen mit Molekularmassen von 200000 bis 1000000, die alleine oder in Form einer Mischung in einem Lösungsmittel verwendet werden;
(d) Harzprodukten aus Siliconen, aufgebaut aus Einheiten: R'₂SiO₂/₂, R'SiO_{3/2}, SiO_{4/2},
in denen R' eine Kohlenwassserstoffgruppe mit 1 bis 6 Kohlenstoffatomen oder eine Phenylgruppe darstellt;
(e) organomodifizierten Siliconen, ausgewählt aus Siliconen, die in ihrer Struktur eine oder mehrere organofunktionelle Gruppierungen aufweisen, die direkt oder über das Zwischenglied eines Kohlenwasserstoffrests an die Siloxan-Kette gebunden sind.

7. Zusammensetzung gemäß Anspruch 6,
**dadurch gekennzeichnet, daß**
die Gummiprodukte aus Silicon, die alleine oder in Form einer Mischung verwendet werden, aus Siliconen folgender Strukturen:
- Poly((dimethylsiloxan)/(methylvinylsiloxan)),
- Poly((dimethylsiloxan)/(diphenylsiloxan)),
- Poly((dimethylsiloxan)/(phenylmethylsiloxan)),
- Poly((dimethylsiloxan)/(diphenylsiloxan)/(methylvinylsiloxan))
und aus folgenden Mischungen ausgewählt sind:
- Mischungen, die aus einem am Kettenende hydroxylierten polydimethylsiloxan und einem cyclischen Polydimethylsiloxan gebildet sind;
- Mischungen, die aus einem Polydimethylsiloxan-Gummi und einem cyclischen Silicon gebildet sind; und
- Mischungen aus Polydimethylsiloxanen unterschiedlicher Viskositäten.

8. Zusammensetzung gemäß Anspruch 6,
**dadurch gekennzeichnet, daß**
die organomodifizierten Silicone aus Polyorganosiloxanen ausgewählt sind, die aufweisen:
a) Polyethylenoxy- und/oder Polypropylenoxy-Gruppierungen,
b) aminierte Gruppierungen, substituiert oder nicht,
c) Thiol-Gruppierungen,
d) Carboxylat-Gruppierungen,
e) alkoxylierte Gruppierungen,
f) Hydroxyalkyl-Gruppierungen,
g) Acyloxyalkyl-Gruppierungen,
h) Alkylcarboxyl-Gruppierungen,
i) 2-Hydroxyalkylsulfonat-Gruppierungen,
j) 2-Hydroxyakylthiosulfat-Gruppierungen und
k) Hydroxacylamino-Grupierungen.

9. Zusammensetzung gemäß jedem der Ansprüche 2 bis 8,
**dadurch gekennzeichnet, daß**
die Polyorganosiloxane aus linearen Polyalkylsiloxanen mit endständigen Trimethylsilylgruppierungen einer Viskosität bei 25°C von 0,2 bis 2,5 m²/s, aus Mischungen eines am Kettenende hydroxylierten Polydimethylsiloxans und einem cyclischen Polydimethylsiloxan, aus Mischungen aus 2 PDMS-Produkten unterschiedlicher Viskositäten aus einem Gummiprodukt und einem Öl sowie aus einem mit Hydroxyacylamino-Gruppierungen modifizierten Polyorganosiloxan ausgewählt sind.

10. Zusammensetzung gemäß jedem der Ansprüche 1 bis 9,
**dadurch gekennzeichnet, daß**
das aus einer Diallyldialkylammoniumverbindung und einem anionischen Monomer abgeleitete Polymer aus einem Polymer zusammengesetzt ist, das 60 bis 99 Gew.% des genannten Polymer an Einheiten aus einem Monomer einer quaternären Diallyldialkylammoniumverbindung enthält, in der die Alkylgruppen, unabhängig voneinander, 1 bis 18 Kohlenstoffatome aufweisen und das entsprechende Anion aus einer Säure mit einer Ionisierungskonstante von mehr als 10-¹³ stammt, wobei ca. 1 bis 40 Gew.% des genannten Polymer aus Einheiten eines anionischen Monomer zusammengesetzt sind, das aus Acryl- und Methacrylsäure ausgewählt ist.

11. Zusammensetzung gemäß Anspruch 10,
**dadurch gekennzeichnet, daß**
das Molekulargewicht des genannten Polymer 50000 bis 10000000 beträgt, bestimmt durch Gelpermeationschromatographie.

12. Zusammensetzung gemäß Anspruch 10 oder 11,
**dadurch gekennzeichnet, daß**
die Alkylgruppen 1 bis 4 Kohlenstoffatome aufweisen.

13. Zusammensetzung gemäß jedem der Ansprüche 10 bis 12,
**dadurch gekennzeichnet, daß**
das Polymer aus Copolymeren von Dimethyldiallylammonium- oder Diethyldiallylammoniumchlorid und Acrylsäure ausgewählt ist.

14. Zusammensetzung gemäß jedem der Ansprüche 1 bis 13,
**dadurch gekennzeichnet, daß**
die oberflächenaktiven Mittel, die reinigende Eigenschaften aufweisen, aus anionischen, amphoteren, zwitterionischen und nicht-ionischen oberflächenaktiven Mitteln oder aus deren Mischungen ausgewählt sind.

15. Zusammensetzung gemäß jedem der Ansprüche 1 bis 14,
**dadurch gekennzeichnet, daß**
das oberflächenaktive Mittel mit reinigenden Eigenschaften in den Zusammensetzungen in Mengenanteilen von 5 bis 50 und insbesondere von 8 bis 35 Gew.% vorhanden ist, bezogen auf das Gesamtgewicht der Zusammensetzung.

16. Zusammensetzung gemäß jedem der Ansprüche 1 bis 15,
**dadurch gekennzeichnet, daß**
das Silicon in Mengenanteilen von 0,2 bis 30 und vorzugsweise von 0,4 bis 15 Gew.% vorhanden ist, bezogen auf das Gesamtgewicht der Zusammensetzung.

17. Zusammensetzung gemäß jedem der Ansprüche 1 bis 16,
**dadurch gekennzeichnet, daß**
das Polymer aus Diallyldialkylammoniumverbindung und anionischem Monomer in Mengenanteilen von 0,1 bis 10 und vorzugsweise von 0,5 bis 5 Gew.% vorhanden ist, bezogen auf das Gesamtgewicht der Zusammensetzung.

18. Zusammensetzung gemäß jedem der Ansprüche 1 bis 17,
**dadurch gekennzeichnet, daß**
das wässrige Milieu aus Wasser oder aus einer Mischung aus Wasser und einem kosmetisch geeigneten Lösungsmittel zusammengesetzt ist.

19. Zusammensetzung gemäß jedem der Ansprüche 1 bis 18,
**dadurch gekennzeichnet, daß**
die Zusammensetzung auch ein Mittel zur Suspendierung enthält, das sich von einem Alkohol unterscheidet, der 27 bis 44 Kohlenstoffatome aufweist und eine bis zwei Etherund/oder Thioether- oder Sulfoxidgruppierungen enthält.

20. Zusammensetzung gemäß jedem der Ansprüche 1 bis 19,
**dadurch gekennzeichnet, daß**
das Suspendiermittel ausgewählt ist aus:
a)
RX (III)
worin R ein aliphatischer Rest mit langer Kohlenstoffkette, die gegebenenfalls durch Sauerstoffatome unterbrochen ist, und X der Rest einer Carboxyl-, Schwefel- oder Phosphorsäure oder ein von einer Carboxylsäure oder einem Amid abgeleiteter Rest sind, wobei die Verbindungen der Formel (III) aus denjenigen ausgewählt sind, in denen gilt:
(i) R ist ein C₁₁₋₂₁-Alkyl- oder -Alkenylrest, X ist:
- eine Gruppierung COOA, worin A ein von einem C₂₋₃-Polyol abgeleiteter Mono- oder Polyhydroxyalkylrest oder ein Rest CH₂CH₂SO₃M ist,
- eine Gruppierung CO(OCH₂CH₂)ₙ-OH, worin n ein Wert von 2 bis 150 ist,
- eine Gruppierung COOCH₂-CH-CH₃-(OCH₂CH₂)ₙOH,
worin n ein Wert von 2 bis 150 ist, wobei die freien OH-Funktionen der oben definierten Gruppierungen mit einer Säure RCOOH verestert sein können, worin R ein C₁₁₋₂₁-Alkyl- oder -Alkenylrest ist,
- eine Gruppierung CONR₁R₂, worin R₁ und R₂ Wasserstoff oder einen Hydroxy-C₁₋₄-alkylrest darstellen, wobei mindestens einer einen Hydroxy-C₁₋₄-alkylrest darstellt,
- eine Gruppierung OSO₃M oder 1/3PO₄³-M₃, worin M ein Alkylimetall, einen Ammonium- oder C₁₋₄-Alkanolamin-Rest darstellt;
(ii) R bedeutet einen Rest R₃(OC₂H₄)ₗOCH₂, und X bedeutet eine Gruppierung COOM, worin M die oben angegebene Bedeutung hat, wobei R₃ einen C₁₂₋₁₄-Alkylrest bedeutet und l eine ganze Zahl oder Dezimalzahl von 2,5 bis 10 ist, oder wobei R₃ auch einen Oleylrest bedeutet und l 2 bis 9 beträgt, oder wobei R₃ auch einen C₈₋₉-Alkylphenylrest bedeutet und l 4 bis 8 beträgt,
oder das Suspendiermittel ist aus Derivaten von RX ausgewählt, in denen R eine C₁₂₋₁₆-Alkylether-Gruppierung und X eine Gruppierung CONR₁R₂ bedeuten, in welcher R₁ und R₂ dieselbe Bedeutung haben, wie sie für die entsprechende Gruppierung oben angegeben ist;
b) Dimethyl-C₁₆₋₂₂-alkylaminoxiden;
c) Biopolysacchariden.

21. Zusammensetzung gemäß einem der Ansprüche 19 oder 20,
**dadurch gekennzeichnet, daß**
das Mittel zur Suspendierung in Mengenanteilen von 0,1 bis 20 Gew.% vorhanden ist, bezogen auf das Gesamtgewicht der Zusammensetzung.

22. Zusammensetzung gemäß jedem der Ansprüche 1 bis 21,
**dadurch gekennzeichnet, daß**
sie ausserdem einen Hilfsstoff, ausgewählt aus Perlmuttglanzmitteln oder opak machenden Mitteln, die in Mengenanteilen bis zu 3% des Gesamtgewichts der Zusammensetzung vorhanden sind, und Viskositätsreguliermittel enthält, die aus Elektrolyten, Hydrotropen und Verdickungsmitteln ausgewählt und in Mengenanteilen bis zu 10 Gew.% vorhanden sind, bezogen auf das Gesamtgewicht der Zusammensetzung.

23. Zusammensetzung gemäß jedem der Ansprüche 1 bis 22,
**dadurch gekennzeichnet, daß**
sie ausserdem Mittel, die die Wirkung aufweisen, die kosmetischen Eigenschaften der Haare und/oder der Haut zu verbessern, und aus kationischen oberflächenaktiven Mitteln und aus Polymeren ausgewählt sind, die sich von den Copolymeren einer Diallyldiammoniumverbindung und eines anionischen Monomer unterscheiden, sowie Proteine oder in dem Milieu lösliche Silicone enthält.

24. Verfahren zum Waschen und/oder Konditionieren keratinischer Materialien,
**dadurch gekennzeichnet, daß**
man auf diese Materialien mindestens eine in jedem der Ansprüche 1 bis 23 definierte Zusammensetzung aufbringt und sodann eine Spülung durchführt.

25. Verwendung einer in jedem der Ansprüche 1 bis 23 definierten Zusammensetzung zum Waschen der Haare.

26. Verwendung einer in jedem der Ansprüche 1 bis 23 definierten Zusammensetzung zum Waschen der Haut.

27. Verfahren zur Herstellung einer Zusammensetzung zum Waschen und/oder Konditionieren keratinischer Materialien, **dadurch gekennzeichnet, daß**
man in ein wässriges Milieu aus Wasser oder aus einer Mischung aus Wasser und einem kosmetisch geeigneten Lösungsmittel einbringt:
a) mindestens ein oberflächenaktives Mittel, ausgewählt aus anionischen, amphoteren, zwitterionischen oder nicht-ionischen oberflächenaktiven Mitteln oder aus deren Mischungen, und zwar in Mengenanteilen, die ausreichen, um der Zusammensetzung eine reinigende Eigenschaft zu verleihen, wobei die Mengenanteile 5 bis 50 und insbesondere 8 bis 35 Gew.% ausmachen, bezogen auf das Gesamtgewicht der Zusammensetzung;
b) mindestens ein in dem angewandten wässrigen Milieu unlösliches Silicon, und zwar in Mengenanteilen von 0,2 bis 30 und vorzugsweise von 0,4 bis 15 Gew.%, bezogen auf das Gesamtgewicht der Zusammensetzung; und
c) mindestens ein Copolymer aus einer Diallyldialkylammoniumverbindung und einem anionischen Monomer, und zwar in Mengenanteilen von 0,1 bis 10 und vorzugsweise von 0,5 bis 5 Gew.%, bezogen auf das Gesamtgewicht der Zusammensetzung; und daß man gegebenenfalls zufügt:
d) 0,1 bis 20 Gew.%, bezogen auf das Gesamtgewicht der Zusammensetzung, eines Mittels zur Suspendierung, das sich von einem Alkohol unterscheidet, der ein C₂₇₋₄₄-Alkohol ist und eine oder zwei Ether- und/oder Thioether- oder Sulfoxidgruppierungen enthält;
e) bis zu 3% des Gesamtgewichts der Zusammensetzung an Perlmuttglanzmitteln oder opak machenden Mitteln;
f) bis zu 10% des Gesamtgewichts der Zusammensetzung an Viskositätsreguliermitteln,
g) weitere Hilfsstoffe, ausgewählt aus Polymeren, die sich von den Copolymeren aus Diallyldialkylammoniumverbindung und anionischem Monomer unterscheiden, aus kationischen oberflächenaktiven Mitteln, Proteinen, im Milieu löslichen Siliconen, Parfüm-Produkten, Konservierungsstoffen, Sequestriermitteln, Schaumstabilisatoren, Treibmitteln, Farbstoffen, Sonnenfilterstoffen und aus sauer oder alkalisch stellenden Mitteln.

## Claims (Claims for the following Contracting State(s): AT, BE, CH, DE, FR, GB, GR, IT, LI, NL, PT, SE, DK)

1. Composition for washing and/or conditioning keratinous matter, **characterized in that** it contains, in an aqueous medium, a surface-active agent possessing detergent properties, at least one silicone which is insoluble in the medium and at least one copolymer derived from diallyldialkylammonium and from an anionic monomer; and not containing dispersing agents composed of alcohols having 27 to 44 carbon atoms and containing one or two ether and/or thioether or sulphoxide groups; the surface-active agent possessing detergent properties being present in the compositions in proportions of between 5 and 50% by weight relative to the total weight of the composition.

2. Composition according to Claim 1, **characterized in that** the silicone is chosen from polyorganosiloxanes which are insoluble in the aqueous medium and is in the form of oil, wax, gum or resin.

3. Composition according to Claim 2, **characterized in that** the polyorganosiloxanes are chosen from volatile silicones.

4. Composition according to Claim 3, **characterized in that** the volatile silicones are chosen from:
- cyclic silicones containing from 3 to 7 silicon atoms;
- cyclopolymers of the dimethylsiloxane/methylalkylsiloxane type;
- mixtures of cyclic silicones with organic compounds derived from silicon;
- linear volatile silicones containing 2 to 9 silicon atoms and with a viscosity lower than or equal to 5×10⁻⁶ m²/s at 25°C.

5. Composition according to Claim 2, **characterized in that** the polyorganosiloxanes are chosen from nonvolatile silicones comprising polyalkylsiloxanes, polyalkylarylsiloxanes, polyarylsiloxanes, silicone gums and resins, organomodified polysiloxanes and mixtures thereof.

6. Composition according to Claim 5, **characterized in that** the nonvolatile polyorganosiloxanes are chosen from:
(a) polyalkylsiloxanes, chosen from:
- linear polydimethylsiloxanes containing trimethylsilyl end groups, with a viscosity of between 5×10⁻⁶ and 2.5 m²/s at 25°C;
- linear polydimethylsiloxanes containing dimethylsilanol end groups;
- poly-C₁-C₂₀-alkylsiloxanes;
(b) polyalkylarylsiloxanes, chosen from:
- polydimethylmethylphenylsiloxanes, polymethylphenylsiloxanes, polydimethyldiphenylsiloxanes which are linear and/or branched, with a viscosity of between 1×10⁻⁵ and 5×10⁻² m²/s at 25°C;
(c) silicone gums chosen from polydiorganosiloxanes which have molecular masses of between 200,000 and 1,000,000, employed by themselves or in the form of mixture in a solvent;
(d) silicone resins, consisting of units:
R'₂SiO_{2/2}, R'SiO_{3/2}, SiO_{4/2}
in which R' denotes a hydrocarbon group containing from 1 to 6 carbon atoms or a phenyl group;
(e) organomodified silicones chosen from silicones whose structure includes one or more organofunctional groups directly attached to the siloxane chain or attached via a hydrocarbon radical.

7. Composition according to Claim 6, **characterized in that** the silicone gums employed by themselves or in the form of a mixture are chosen from the silicones of following structures:
- poly[(dimethylsiloxane)/(methylvinylsiloxane)],
- poly[(dimethylsiloxane)/(diphenylsiloxane)],
- poly[(dimethylsiloxane)/(phenylmethylsiloxane)],
- poly[(dimethylsiloxane)/(diphenylsiloxane)/(methylvinylsiloxane)]
and the following mixtures: .
- mixtures made up from a polydimethylsiloxane hydroxylated at the end of a chain and a cyclic polydimethylsiloxane;
- the mixtures made up from a polydimethylsiloxane gum and from a cyclic silicone; and
- mixtures of polydimethylsiloxanes of different viscosities.

8. Composition according to Claim 6, **characterized in that** the organomodified silicones are chosen from the polyorganosiloxanes containing:
a)polyethyleneoxy and/or polypropyleneoxy groups;
b) substituted or unsubstituted amine groups;
c) thiol groups;
d) carboxylate groups;
e) alkoxy groups;
f) hydroxyalkyl groups;
g) acyloxyalkyl groups;
h) alkylcarboxylic groups;
i) 2-hydroxyalkylsulphonate groups;
j) 2-hydroxyalkylthiosulphate groups;
k) hydroxyacylamino groups.

9. Composition according to any one of Claims 2 to 8, **characterized in that** the polyorganosiloxanes are chosen from linear polyalkylsiloxanes containing trimethylsilyl end groups, with a viscosity of between 0.2 and 2.5 m²/s at 25°C, mixtures of a polydimethylsiloxane hydroxylated at the end of a chain and of a cyclic polydimethylsiloxane, mixtures of two PDMSs consisting of a gum and an oil of different viscosities, and a polyorganosiloxane modified with hydroxyacylamino groups.

10. Composition according to any one of Claims 1 to 9, **characterized in that** the polymer derived from diallyldialkylammonium and from an anionic monomer consists of a polymer containing 60 to 99 % by weight of the said polymer of units derived from a quaternary diallyldialkylammonium monomer in which the alkyl groups have, independently from each other, 1 to 18 carbon atoms, the corresponding anion being derived from an acid which has an ionization constant higher than 10⁻¹³ and approximately 1 to 40 % by weight of the said polymer consisting of units of an anionic monomer chosen from acrylic acid and methacrylic acid.

11. Composition according to Claim 10, **characterized in that** the molecular weight of the said polymer is between 50,000 and 10,000,000 determined by gel permeation chromatography.

12. Composition according to Claim 10 or 11, **characterized in that** the alkyl groups have between 1 and 4 carbon atoms.

13. Composition according to any one of Claims 10 to 12, **characterized in that** the polymer is chosen from copolymers of dimethyldiallylammonium or diethyldiallylammonium chloride and of acrylic acid.

14. Composition according to any one of Claims 1 to 13, **characterized in that** the surface-active agents are chosen from anionic, amphoteric, zwitterionic or nonionic surface-active agents or mixtures thereof which have detergent properties.

15. Composition according to any one of Claims 1 to 14, **characterized in that** the surface-active agent with detergent properties is present in the compositions in proportions of between 8 and 35 % by weight relative to the total weight of the composition.

16. Composition according to any one of Claims 1 to 15, **characterized in that** the silicone is present in proportions of between 0.2 and 30 % by weight, and preferably between 0.4 and 15 % by weight relative to the total weight of the composition.

17. Composition according to any one of Claims 1 to 16, **characterized in that** the polymer of diallyldialkylammonium and of an anionic monomer is present in proportions of between 0.1 and 10 % by weight relative to the total weight of the composition, and preferably between 0.5 and 5 % by weight relative to the total weight of the composition.

18. Composition according to any one of Claims 1 to 17, **characterized in that** the aqueous medium consists of water or a mixture of water and of a cosmetically acceptable solvent.

19. Composition according to any one of Claims 1 to 18, **characterized in that** the composition also contains a dispersing agent other than an alcohol, containing 27 to 44 carbon atoms and containing one to two ether and/or thioether or sulphoxide groups.

20. Composition according to any one of Claims 1 to 19, **characterized in that** the dispersing agent is chosen from:
a)
RX (III)
in which R is an aliphatic radical containing a long carbon chain optionally interrupted by oxygen atoms, and X is a carboxylic, sulphuric or phosphoric acid residue or a radical derived from a carboxylic acid or from an amide; these compounds of formula (III) are chosen from those in which:
(i) R is a C₁₁-C₂₁ alkyl or alkenyl radical
X is:
- a group COOA where A is a mono- or polyhydroxyalkyl radical derived from a C₂-C₃ polyol or a radical CH₂CH₂SO₃M,
- a group CO(OCH₂CH₂)ₙ-OH where n has a value of between 2 and 150,
- a group 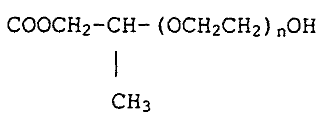 where n has a value of between 2 and 150, it being possible for the free OH functional groups of the groups defined above to be esterified with an acid RCOOH where R is a C₁₁-C₂₁ alkyl or alkenyl,
- a group CONR₁R₂ where R₁ and R₂ denote hydrogen or C₁-C₄ hydroxyalkyl, at least one denoting C₁-C₄ hydroxyalkyl,
- a group OSO₃M or 1/3 PO₄³⁻M₃ where M denotes an alkali metal, ammonium or a C₁-C₄ alkanolamine residue;
(ii) R denotes a radical R₃ (OC₂H₄)ₗOCH₂ and X denotes a group COOM where M has the meaning indicated above, R₃ denoting a C₁₂-C₁₄ alkyl radical and *l* a whole or decimal number between 2.5 and 10, or else R₃ denotes oleyl and *l* varies from 2 to 9, or else R₃ denotes C₈-C₉-alkylphenyl and *l* varies from 4 to 8, or the derivatives in which R denotes a C₁₂-C₁₆ alkyl ether group and X a group CONR₁R₂ in which R₁ and R₂ have the same meaning as that indicated above;
b) oxides of dirnethyl-C₁₆-C₂₂-alkylamines;
c) biopolysaccharides.

21. Composition according to either of Claims 19 or 20, **characterized in that** the dispersing agent is present in proportions of between 0.1 and 20 % by weight of the total weight of the composition.

22. Composition according to any one of Claims 1 to 21, **characterized in that** it additionally contains an adjuvant chosen from lustering or opacifying agents present in proportions ranging up to 3 % of the total weight of the composition, viscosity modifiers chosen from electrolytes, hydrotropic agents and thickening agents present in proportions ranging up to 10 % by weight relative to the total weight of the composition.

23. Composition according to any one of Claims 1 to 22, **characterized in that** it additionally contains agents whose effect is to improve the cosmetic properties of hair and/or of the skin, which are chosen from cationic surface-active agents, polymers other than the copolymers of diallyldialkylammonium and of an anionic monomer, proteins or silicones which are soluble in the mixture.

24. Process for washing and/or conditioning keratinous matter, **characterized in that** at least one composition as defined in any one of Claims 1 to 23 is applied to this matter and that a rinsing is then carried out.

25. Use of a composition as defined in any one of Claims 1 to 23 for washing hair.

26. Use of a composition as defined in any one of Claims 1 to 23 for washing the skin.

## Claims (Claims for the following Contracting State(s): ES)

1. Composition for washing and/or conditioning keratinous matter, **characterized in that** it contains, in an aqueous medium, a surface-active agent possessing detergent properties, at least one silicone which is insoluble in the medium and at least one copolymer derived from diallyldialkylammonium and from an anionic monomer; and riot containing dispersing agents composed of alcohols having 27 to 44 carbon atoms and containing one or two ether and/or thioether or sulphoxide groups; the surface-active agent possessing detergent properties being present in the compositions in proportions of between 5 and 50% by weight relative to the total weight of the composition.

2. Composition according to Claim 1, **characterized in that** the silicone is chosen from polyorganosiloxanes which are insoluble in the aqueous medium and is in the form of oil, wax, gum or resin.

3. Composition according to Claim 2, **characterized in that** the polyorganosiloxanes are chosen from volatile silicones.

4. Composition according to Claim 3, **characterized in that** the volatile silicones are chosen from:
- cyclic silicones containing from 3 to 7 silicon atoms;
- cyclopolymers of the dimethylsiloxane/methylalkylsiloxane type;
- mixtures of cyclic silicones with organic compounds derived from silicon;
- linear volatile silicones containing 2 to 9 silicon atoms and with a viscosity lower than or equal to 5×10⁻⁶ m²/s at 25°C.

5. Composition according to Claim 2, **characterized in that** the polyorganosiloxanes are chosen from nonvolatile silicones comprising polyalkylsiloxanes, polyalkylarylsiloxanes, polyarylsiloxanes, silicone gums and resins, organomodified polysiloxanes and mixtures thereof.

6. Composition according to Claim 5, **characterized in that** the nonvolatile polyorganosiloxanes are chosen from:
(a) polyalkylsiloxanes, chosen from:
- linear polydimethylsiloxanes containing trimethylsilyl end groups, with a viscosity of between 5×10⁻⁶ and 2.5 m²/s at 25°C;
- linear polydimethylsiloxanes containing dimethylsilanol end groups;
- poly-C₁-C₂₀-alkylsiloxanes;
(b) polyalkylarylsiloxanes, chosen from:
- polydimethylmethylphenylsiloxanes, polymethylphenylsiloxanes, polydimethyldiphenylsiloxanes which are linear and/or branched, with a viscosity of between 1×10-⁵ and 5×10⁻² m²/s at 25°C;
(c) silicone gums chosen from polydiorganosiloxanes which have molecular masses of between 200,000 and 1,000,000, employed by themselves or in the form of mixture in a solvent;
(d) silicone resins, consisting of units:
R'₂SiO_{2/2}, R'SiO_{3/2}, SiO_{4/2}
in which R' denotes a hydrocarbon group containing from 1 to 6 carbon atoms or a phenyl group;
(e) organomodified silicones chosen from silicones whose structure includes one or more organofunctional groups directly attached to the siloxane chain or attached via a hydrocarbon radical.

7. Composition according to Claim 6, **characterized in that** the silicone gums employed by themselves or in the form of a mixture are chosen from the silicones of following structures:
- poly[(dimethylsiloxane)/(methylvinylsiloxane)],
- poly[(dimethylsiloxane)/(diphenylsiloxane)],
- poly[(dimethylsiloxane)/(phenylmethylsiloxane)],
- poly[(dimethylsiloxane)/(diphenylsiloxane)/(methyl-vinylsiloxane)]
and the following mixtures:
- mixtures made up from a polydimethylsiloxane hydroxylated at the end of a chain and a cyclic polydimethylsiloxane;
- the mixtures made up from a polydimethylsiloxane gum and from a cyclic silicone; and
- mixtures of polydimethylsiloxanes of different viscosities.

8. Composition according to Claim 6, **characterized in that** the organomodified silicones are chosen from the polyorganosiloxanes containing:
'a)polyethyleneoxy and/or polypropyleneoxy groups;
b) substituted or unsubstituted amine groups;
c) thiol groups;
d) carboxylate groups;
e) alkoxy groups;
f) hydroxyalkyl groups;
g) acyloxyalkyl groups;
h) alkylcarboxylic groups;
i) 2-hydroxyalkylsulphonate groups;
j) 2-hydroxyalkylthiosulphate groups;
k) hydroxyacylamino groups.

9. Composition according to any one of Claims 2 to 8, **characterized in that** the polyorganosiloxanes are chosen from linear polyalkylsiloxanes containing trimethylsilyl end groups, with a viscosity of between 0.2 and 2.5 m²/s at 25°C, mixtures of a polydimethylsiloxane hydroxylated at the end of a chain and of a cyclic polydimethylsiloxane, mixtures of two PDMSs consisting of a gum and an oil of different viscosities, and a polyorganosiloxane modified with hydroxyacylamino groups.

10. Composition according to any one of Claims 1 to 9, **characterized in that** the polymer derived from diallyldialkylammonium and from an anionic monomer consists of a polymer containing 60 to 99 % by weight of the said polymer of units derived from a quaternary diallyldialkylammonium monomer in which the alkyl groups have, independently from each other, 1 to 18 carbon atoms, the corresponding anion being derived from an acid which has an ionization constant higher than 10⁻¹³ and approximately 1 to 40 % by weight of the said polymer consisting of units of an anionic monomer chosen from acrylic acid and methacrylic acid.

11. Composition according to Claim 10, **characterized in that** the molecular weight of the said polymer is between 50,000 and 10,000,000 determined by gel permeation chromatography.

12. Composition according to Claim 10 or 11, **characterized in that** the alkyl groups have between 1 and 4 carbon atoms.

13. Composition according to any one of Claims 10 to 12, **characterized in that** the polymer is chosen from copolymers of dimethyldiallylammonium or diethyldiallylammonium chloride and of acrylic acid.

14. Composition according to any one of Claims 1 to 13, **characterized in that** the surface-active agents are chosen from anionic, amphoteric, zwitterionic or nonionic surface-active agents or mixtures thereof which have detergent properties.

15. Composition according to any one of Claims 1 to 14, **characterized in that** the surface-active agent with detergent properties is present in the compositions in proportions of between 8 and 35 % by weight relative to the total weight of the composition.

16. Composition according to any one of Claims 1 to 15, **characterized in that** the silicone is present in proportions of between 0.2 and 30 % by weight, and preferably between 0.4 and 15 % by weight relative to the total weight of the composition.

17. Composition according to any one of Claims 1 to 16, **characterized in that** the polymer of diallyldialkylammonium and of an anionic monomer is present in proportions of between 0.1 and 10 % by weight relative to the total weight of the composition, and preferably between 0.5 and 5 % by weight relative to the total weight of the composition.

18. Composition according to any one of Claims 1 to 17, **characterized in that** the aqueous medium consists of water or a mixture of water and of a cosmetically acceptable solvent.

19. Composition according to any one of Claims 1 to 18, **characterized in that** the composition also contains a dispersing agent other than an alcohol, containing 27 to 44 carbon atoms and containing one to two ether and/or thioether or sulphoxide groups.

20. Composition according to any one of Claims 1 to 19, **characterized in that** the dispersing agent is chosen from:
a)
RX (III)
in which R is an aliphatic radical containing a long carbon chain optionally interrupted by oxygen atoms, and X is a carboxylic, sulphuric or phosphoric acid residue or a radical derived from a carboxylic acid or from an amide; these compounds of formula (III) are chosen from those in which:
(i) R is a C₁₁-C₂₁ alkyl or alkenyl radical
X is:
- a group COOA where A is a mono- or polyhydroxyalkyl radical derived from a C₂-C₃ polyol or a radical CH₂CH₂SO₃M,
- a group CO (OCH₂CH₂) ₙ-OH where n has a value of between 2 and 150,
- a group 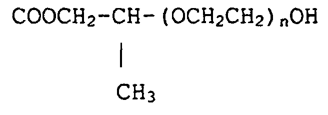 where n has a value of between 2 and 150, it being possible for the free OH functional groups of the groups defined above to be esterified with an acid RCOOH where R is a C₁₁-C₂₁ alkyl or alkenyl,
- a group CONR₁R₂ where R₁ and R₂ denote hydrogen or C₁-C₄ hydroxyalkyl, at least one denoting C₁-C₄ hydroxyalkyl,
- a group OSO₃M or 1/3 PO₄³⁻M₃ where M denotes an alkali metal, ammonium or a C₁-C₄ alkanolamine residue;
(ii) R denotes a radical R₃(OC₂H₄)_{*l*}OCH₂ and X denotes a group COOM where M has the meaning indicated above, R₃ denoting a C₁₂-C₁₄ alkyl radical and *l* a whole or decimal number between 2.5 and 10, or else R₃ denotes oleyl and *l* varies from 2 to 9, or else R₃ denotes C₈-C₉-alkylphenyl and *l* varies from 4 to 8, or the derivatives in which R denotes a C₁₂-C₁₆ alkyl ether group and X a group CONR₁R₂ in which R₁ and R₂ have the same meaning as that indicated above;
b) oxides of dimethyl-C₁₆-C₂₂-alkylamines;
c) biopolysaccharides.

21. Composition according to either of Claims 19 or 20, **characterized in that** the dispersing agent is present in proportions of between 0.1 and 20 % by weight of the total weight of the composition.

22. Composition according to any one of Claims 1 to 21, **characterized in that** it additionally contains an adjuvant chosen from lustering or opacifying agents present in proportions ranging up to 3 % of the total weight of the composition, viscosity modifiers chosen from electrolytes, hydrotropic agents and thickening agents present in proportions ranging up to 10 % by weight relative to the total weight of the composition.

23. Composition according to any one of Claims 1 to 22, **characterized in that** it additionally contains agents whose effect is to improve the cosmetic properties of hair and/or of the skin, which are chosen from cationic surface-active agents, polymers other than the copolymers of diallyldialkylammonium and of an anionic monomer, proteins or silicones which are soluble in the mixture.

24. Process for washing and/or conditioning keratinous matter, **characterized in that** at least one composition as defined in any one of Claims 1 to 23 is applied to this matter and that a rinsing is then carried out.

25. Use of a composition as defined in any one of Claims 1 to 23 for washing hair.

26. Use of a composition as defined in any one of Claims 1 to 23 for washing the skin.

27. Process for the preparation of a composition for washing and/or conditioning keratinous matter, **characterized in that** the following are introduced into an aqueous medium consisting of water or a mixture of water and of a cosmetically acceptable solvent:
a) at least one surface-active agent chosen from anionic, amphoteric, zwitterionic and nonionic surface-active agents or mixtures thereof, in proportions which are sufficient to impart a detergent character to the composition and are of between 5 and 50% by weight and in particular between 8 and 35% by weight relative to the total weight of the composition;
b) at least one silicone which is insoluble in the aqueous medium employed, in proportions of between 0.2 and 30% and preferably between 0.4 and 15% by weight relative to the total weight of the composition; and
c) at least one copolymer derived from diallyldialkylammonium and from an anionic monomer in proportions of between 0.1 and 10% and preferably between 0.5 and 5% by weight relative to the total weight of the composition; and **in that** the following are optionally added:
d) between 0.1 and 20% by weight relative to the total weight of the composition of a C₂₇-C₄₄ dispersing agent other than an alcohol, containing one or two ether and/or thioether or sulphoxide groups;
e) up to 3% of the total weight of the composition of lustering or opacifying agents;
f) up to 10% of the total weight of the composition of viscosity modifiers;
g) other adjuvants chosen from polymers other than the copolymers of diallyldialkylammonium and of an anionic monomer, cationic surfactants, proteins, silicones which are soluble in the mixture, perfumes, stabilizers, sequestrants, foam stabilizers, propellants, colorants, sunscreens and acidifying or alkalifying agents.
